# EUROPÄISCHE PATENTSCHRIFT

(11) **EP 3 858 413 B1**
(45) Veröffentlichungstag und Bekanntmachung des Hinweises auf die Patenterteilung: **16.02.2022**
(21) Anmeldenummer: 21152650.4
(22) Anmeldetag: 21.01.2021
(51) Int. Cl.: A61M 16/08, A61M 16/00, A61M 16/04, A61M 39/10, A61M 25/01, B29C 64/00, B33Y 80/00, A61M 16/01, A61M 16/06, A61M 16/10

(54) **GEKRÜMMTE VERBINDUNGSEINHEIT ZUM VERBINDEN EINER PATIENTENSEITIGEN KOPPLUNGSEINHEIT MIT EINEM MEDIZINISCHEN GERÄT**
CURVED CONNECTING UNIT FOR CONNECTING A PATIENT SIDE COUPLING UNIT TO A MEDICAL APPARATUS
UNITÉ DE LIAISON COURBÉE DESTINÉE À LA LIAISON D'UNE UNITÉ D'ACCOUPLEMENT DU CÔTÉ D'UN PATIENT À UN APPAREIL MÉDICAL

(30) Priorität: 28.01.2020 DE 102020000503
(43) Veröffentlichungstag der Anmeldung: 04.08.2021
(73) Patentinhaber: Drägerwerk AG & Co. KGaA, 23558 Lübeck (DE)
(72) Erfinder: Tappehorn, Ludger, 23558 Lübeck (DE); Hoder, Matthias, 23558 Lübeck (DE); Lütkhoff, Jan-Henning, 23558 Lübeck (DE)

(56) Entgegenhaltungen:
- WO-A1-96/26757
- WO-A1-2014/205513
- WO-A1-2018/236228
- DE-A1-102008 022 987
- DE-A1-102016 119 707
- US-A- 5 156 159
- US-A- 5 694 922
- US-A1- 2004 221 852
- US-A1- 2010 147 296
- US-A1- 2011 067 699
- US-A1- 2016 051 792
- US-A1- 2016 310 688
- US-A1- 2017 258 550
- US-A1- 2019 240 435
- US-B2- 9 295 805
- US-B2- 10 413 687

## Beschreibung

Die Erfindung betrifft eine Verbindungseinheit, mit deren Hilfe sich eine patientenseitige Kopplungseinheit mit einem medizinischen Gerät, wobei das medizinische Gerät ein Beatmungsgerät oder ein Anästhesiegerät ist, verbinden lässt, wobei die Verbindungseinheit die Strömungsrichtung eines Fluids verändert und die Einführung eines intrakorporalen Geräts ermöglicht. Weiterhin betrifft die Erfindung eine Verwendung einer solchen Verbindungseinheit sowie ein Verfahren zur Herstellung einer solchen Verbindungseinheit.

Verschiedene derartige Verbindungseinheiten sind bekannt geworden.

In US 5,694,922 wird eine Verbindungseinheit (adaptor) beschrieben, welche einen Patienten mit Schläuchen (exterior tubing) zu verbinden vermag. Die L-förmige Verbindungseinheit 44 besitzt eine Öffnung, durch die hindurch sich ein weiteres Gerät einführen lässt.

US 2004 / 0 221 852 A1 zeigt eine Beatmungsvorrichtung (respiratory apparatus 10) mit einem Katheter (suction catheter 12), der sich in den Atemweg eines Patienten 18 legen lässt, um Sekrete abzusaugen. Der Katheter 12 lässt sich durch ein Einführstück (instrument introduction section 22 with einem passageway 24) hindurch einführen.

Auch in US 2010 / 0 147 296 A1 wird beschrieben, wie ein Katheter (suction catheter 12) eines Beatmungsgeräts (catheter aspirating device 10) in den Atemweg eines Patienten eingeführt wird. Das Beatmungsgerät 10 lässt sich mit einem Verbindungsstück (multiple-access manifold 20) verbinden, durch das hindurch sich der Katheter 12 einführen lässt. Ein Port 30 des Verbindungsstücks 20 lässt sich mit einem Verschlusselement (port seal cartridge 40) verschließen.

US 2013 / 0 296 653 A1 zeigt ein System 100 zur künstlichen Beatmung mit einer Atemmaske 102, die sich auf den Kopf eines Patienten legen lässt, sowie einem Adapter 110, der sich mit der Atemmaske 102 koppeln lässt. In ein Einführstück (insertion assembly 117) des Adapters 110 lässt sich ein gebogenes Instrument (assisted respiration source assembly 119 with diverter 114) einstecken. Das Einführstück 117 ist lösbar mit einem Verbindungsstück (connection assembly 130) des Adapters 110 verbunden. In das Instrument 119 ist ein Ventil 128, beispielsweise ein Rückschlagventil (one-way valve), eingebaut.

In US 2019 / 0 240 435 A1 wird eine patientenseitige Koppeleinheit (oral apliance 100) beschrieben, die sich mit einem Atemlufterzeuger (continuous positive airway pressure) verbinden lässt. Ein Körper 110, der Koppeleinheit 100 umfasst mindestens eine nach außen zeigende Öffnung (extra-oral opening 111), welche einen Luftstrom durch die Lippen eines Patienten ermöglicht, und eine nach innen zeigende Öffnung (intra-oral opening 121). An den Körper 110 lässt sich eine Verbindungseinheit 200 oder 300 anschließen.

In DE 10 2016 119 707 A1 wird ein Adapter 29 beschrieben, mit dem sich ein Endoskop 1 an ein Aufbereitungsgerät 210 anschließen lässt. Ein System 45 von Verbindungskanälen vermag einen Kanal 7, 8 des Endoskops eins mit einem Kanal 21, 22 des Aufbereitungsgeräts zu verbinden. Wenigstens teilweise ist dieser Adapter 29 in einem 3D-Druckverfahren hergestellt.

Der Erfindung liegt die Aufgabe zugrunde, eine Verbindungseinheit zum Herstellen einer Fluidverbindung zwischen einer patientenseitigen Kopplungseinheit und einem medizinischen Gerät, wobei das medizinische Gerät ein Beatmungsgerät oder ein Anästhesiegerät ist, bereitzustellen, wobei die Verbindungseinheit es ermöglicht, ein weiteres Gerät anzuschließen, und wobei die Gefahr, dass beim Anschließen des weiteren Geräts eine unerwünschte Situation auftritt, geringer als bei bekannten Verbindungseinheiten sein soll. Insbesondere soll ein mit der patientenseitigen Kopplungseinheit verbundener Patient beim Anschließen nicht gefährdet werden.

Die Aufgabe wird durch eine Verbindungseinheit mit den Merkmalen des Anspruchs 1, durch ein Fluidführungbauteil mit den Merkmalen des Anspruchs 9, durch eine Fluidführungs-Anordnung mit den Merkmalen des Anspruchs 10, durch ein Fluidführungssystem mit den Merkmalen des Anspruchs 11, durch eine medizinische Anordnung mit den Merkmalen des Anspruchs 12, durch eine Verwendung mit den Merkmalen des Anspruchs 13, durch ein Verfahren mit den Merkmalen des Anspruchs 14, durch ein Computerprogramm mit den Merkmalen des Anspruchs 15 sowie durch einen 3D-Drucker mit den Merkmalen des Anspruchs 16 gelöst. Vorteilhafte Ausgestaltungen sind in den Unteransprüchen 2 bis 8 angegeben.

Die erfindungsgemäße Verbindungseinheit trägt dazu bei, eine Fluidverbindung zwischen einer patientenseitigen Kopplungseinheit und einem medizinischen Gerät herzustellen. Das medizinische Gerät ist ein Beatmungsgerät oder ein Anästhesiegerät.

Ein Anästhesiegerät ist ein Sonderfall eines Beatmungsgeräts. Die patientenseitige Kopplungseinheit lässt sich lösbar mit einem Patienten verbinden, beispielsweise indem man sie auf sein Gesicht legt oder in seinen Körper einführt, insbesondere in seine Luftröhre oder in seine Speiseröhre. Spezielle Beispiele für patientenseitige Kopplungseinheiten sind eine Atemmaske, ein Endotrachealtubus und ein Messschlauch mit einem Messballon.

Dank dieser Fluidverbindung kann ein Fluid von dem medizinischen Gerät zu der patientenseitigen Kopplungseinheit und damit zu dem Patienten fließen, optional auch in die entgegengesetzte Richtung.

Die patientenseitige Kopplungseinheit ist mit einer patientenseitigen Fluidführungseinheit verbunden oder lässt sich mit einer patientenseitigen Fluidführungseinheit verbinden. Das medizinische Gerät ist mit einer geräteseitigen Fluidführungseinheit verbunden oder lässt sich mit einer geräteseitigen Fluidführungseinheit verbinden.

Die erfindungsgemäße Verbindungseinheit umfasst
- ein hohles patientenseitiges Verbindungsstück,
- ein geräteseitiges Verbindungsstück,
- ein hohles Anschlussstück und
- ein hohles Mittelstück.

Das Anschlussstück umfasst
- ein gerades Röhrensegment und
- ein flächiges Element mit einer gebogenen Oberfläche.

Der Begriff "flächiges Element" bezeichnet ein Element, das sich in einer - in der Regel gebogenen - Fläche erstreckt und dessen Dicke höchstens halb so groß, bevorzugt höchstens ein Viertel, besonders bevorzugt höchstens ein Zehntel so groß ist wie die maximale Ausdehnung des flächigen Elements in der Fläche.

Das flächige Element weist eine Durchführungs-Öffnung auf. Diese Durchführungs-Öffnung ist in die gebogene Oberfläche des flächigen Elements eingelassen. Die Durchführungs-Öffnung nimmt höchstens die Hälfte der Fläche der gebogenen Oberfläche ein.

Das Mittelstück umfasst ein gekrümmtes Röhrensegment mit einer Wand.

Eine patientenseitige Fluidführungseinheit lässt sich an das patientenseitige Verbindungsstück der Verbindungseinheit anschließen oder ist angeschlossen. Mithilfe dieser patientenseitigen Fluidführungseinheit lässt sich eine Fluidverbindung zwischen der Verbindungseinheit und der patientenseitigen Kopplungseinheit herstellen. Dadurch lässt sich auch eine Fluidverbindung zwischen der Verbindungseinheit und dem Patienten herstellen, der mit der patientenseitigen Kopplungseinheit verbunden ist, wobei der Patient medizinisch behandelt, beispielsweise künstlich beatmet, werden soll.

Der Begriff "Fluidführungseinheit" bezeichnet ein Bauteil, das ein Fluid von einem Punkt entlang einer Bahn zu einem anderen Punkt führen kann, ohne dass das Fluid seitlich austritt. Der Begriff "Fluidführungseinheit" umfasst insbesondere einen Schlauch, z.B. einen Faltenschlauch, eine Röhre, z.B. ein röhrenförmiges Verbindungselement oder einen Konnektor, sowie einen Tubus oder Katheter, insbesondere einen Tubus, der sich in die Luftröhre oder Speiseröhre eines Patienten einführen lässt. Der Begriff "Röhre" bezeichnet eine Fluidführungseinheit aus einem relativ starren Material, der Begriff "Schlauch" eine Fluidführungseinheit aus einem biegsamen Material.

Eine geräteseitige Fluidführungseinheit lässt sich an das geräteseitige Verbindungsstück der Verbindungseinheit anschließen oder ist an dieses angeschlossen. Mithilfe dieser geräteseitigen Fluidführungseinheit lässt sich eine Fluidverbindung zwischen der Verbindungseinheit und einem medizinischen Gerät, herstellen. Das medizinische Gerät ist ein Beatmungsgerät, welches den Patienten künstlich beatmen soll, oder ein Anästhesiegerät. Ein

Anästhesiegerät ist ein Sonderfall eines Beatmungsgeräts.

Das Mittelstück umfasst zwei Enden, zwischen denen sich das gekrümmte Röhrensegment befindet. Das eine Ende des Mittelstücks ist fluiddicht mit dem patientenseitigen Verbindungsstück verbunden oder lässt sich fluiddicht verbinden. Das andere Ende des Mittelstücks ist fluiddicht mit dem geräteseitigen Verbindungsstück verbunden oder lässt sich fluiddicht verbinden. Die Verbindungseinheit stellt eine gekrümmte Röhre bereit, welche die beiden hohlen Verbindungsstücke und das gekrümmte Röhrensegment umfasst. Dank der gekrümmten Röhre ändert die Verbindungseinheit die Richtung, in welche ein Fluid durch die Verbindungseinheit fließt.

Ein Fluid kann durch die angeschlossene geräteseitige Fluidführungseinheit, die bereitgestellte gekrümmte Röhre und die angeschlossene patientenseitige Fluidführungseinheit hindurch vom medizinischen Gerät zur patientenseitigen Kopplungseinheit und optional zurück von der patientenseitigen Kopplungseinheit zum medizinischen Gerät fließen. Dadurch kann das Fluid vom medizinischen Gerät zum angeschlossenen Patienten fließen. Die Fließrichtung des Fluids wird im erfindungsgemäßen gekrümmten Röhrensegment geändert, d.h. das Fluid wird umgelenkt.

Das Mittelstück weist eine Aufnahme-Öffnung auf. Das Anschlussstück ist in diese Aufnahme-Öffnung eingesetzt oder lässt sich in diese einsetzen. Wenn das Anschlussstück eingesetzt ist, bildet die gebogene Oberfläche des Anschlussstücks einen Teil der Wand des gekrümmten Röhrensegments des Mittelstücks.

Das Anschlussstück umfasst ein gerades Röhrensegment. Dieses gerade Röhrensegment und das Mittelstück stellen zusammen eine gerade Röhre bereit. Diese gerade Röhre ist durch das flächige Element des eingesetzten Anschlussstücks unterbrochen. Die gerade Röhre führt durch die Durchführungs-Öffnung im flächigen Element und setzt sich im Inneren des patientenseitigen Verbindungsstücks fort.

Die erfindungsgemäße Verbindungseinheit ermöglicht dank der geraden Röhre folgende Verwendung: Ein weiteres Gerät lässt sich in das Innere der geraden Röhre einführen. Diese gerade Röhre wird durch das gerade Röhrensegment und das Mittelstück bereitgestellt. Um das weitere Gerät einzuführen, lässt es sich durch das gerade Röhrensegment und durch die Durchführungs-Öffnung hindurch in das Innere des patientenseitigen Verbindungsstücks hinein einführen und zur patientenseitigen Kopplungseinheit hin bewegen. Weil eine gerade Röhre bereitgestellt wird, kann das weitere Gerät die Form eines starren Stabs oder einer starren Röhre haben. Die Gefahr wird reduziert, dass das weitere Gerät sich an einer Wand eines Bestandteils der Fluidverbindung verhakt oder dass das weitere Gerät oder die Wand beschädigt werden.

Erfindungsgemäß umfasst das Mittelstück ein gekrümmtes Röhrensegment, welches zu der bereitgestellten gekrümmten Röhre gehört oder welches die gekrümmte Röhre ausbildet. Dieses gekrümmte Röhrensegment hat eine gebogene Oberfläche, bevorzugt ohne Kanten und Ecken. Durch dieses Merkmal der gebogenen Oberfläche verursacht die gekrümmte Röhre einen geringeren und / oder weniger von Position zu Position variierenden Strömungswiderstand als andere Verbindungseinheiten, wenn ein Fluid vom medizinischen Gerät durch die gekrümmte Röhre hindurch zum Patienten oder umgekehrt fließt. Der Strömungswiderstand ist dann besonders gering oder variiert dann besonders wenig, wenn die gebogene Oberfläche glatt ist. Die Gefahr, dass Verwirbelungen in der gekrümmten Röhre auftreten, ist geringer verglichen mit einer Ausgestaltung, bei welcher an der Innenwand der Röhre eine Kante auftritt. Solche Verwirbelungen können die Messergebnisse eines Sensors für einen Parameter eines Fluidstroms verfälschen.

Diese beiden Wirkungen, nämlich der geringere und / oder weniger variierende Strömungswiderstand sowie das geringere Ausmaß von Verwirbelungen, erleichtern es, den Druck und / oder den Volumenfluss in einer Fluidverbindung zwischen dem medizinischen Gerät und dem Patienten zu regeln und für diese Regelung einen gewünschten zeitlichen Verlauf des Drucks als Führungsgröße vorzugeben. Eine gute Regelung erleichtert es insbesondere, die künstliche Beatmung eines Patienten allmählich zu reduzieren und dadurch den Patienten von der künstlichen Beatmung zu "entwöhnen". Falls das Beatmungsgerät als Anästhesiegerät ausgestaltet ist, so erleichtert diese Ausgestaltung es, die verwendeten Narkosemittel korrekt zu dosieren.

Erfindungsgemäß lässt sich ein weiteres Gerät durch das Anschlussstück hindurch in das Innere der Röhre einbringen. Dieses weitere Gerät ist beispielsweise ein Katheter, mit dem Sekret endotrachal aus dem Körper des Patienten abgesogen wird, oder eine Sonde oder ein Endoskop.

Erfindungsgemäß stellen das gerade Röhrensegment des Anschlussstücks und das Mittelstück zusammen eine gerade Röhre bereit. Durch diese gerade Röhre hindurch lässt sich das weitere Gerät einführen. Dank dem Anschlussstück der erfindungsgemäßen Verbindungseinheit ist es nicht erforderlich, die Fluidverbindung zwischen dem medizinischen Gerät und der patientenseitigen Kopplungseinheit zu trennen, um das weitere Gerät einzuführen. Eine solche Trennung würde häufig das Ziel der medizinischen Behandlung, z.B. die künstlichen Beatmung, gefährden. Außerdem kostet die Wiederherstellung der Fluidverbindung Zeit. Darüber hinaus müsste an der Trennstelle eine Verschlusseinheit angebracht werden, was zu Verunreinigungen führen kann. Die Erfindung spart also Zeit beim Einführen des weiteren Geräts und reduziert die Gefahr einer Verunreinigung.

Um das weitere Gerät einzuführen, wird bevorzugt eine optionale Kappe oder ein sonstiger Verschluss von dem geraden Röhrensegment des Anschlussstücks entfernt, und das weitere Gerät wird durch das gerade Röhrensegment des Anschlussstücks hindurch bewegt. Der Verschluss lässt sich wieder aufsetzen, wenn das weitere Gerät wieder entfernt worden ist. Der wieder aufgesetzte Verschluss ist vor allem dann vorteilhaft, wenn die Fluidverbindung aufrecht erhalten wird, nachdem das weitere Gerät entfernt worden ist.

Erfindungsgemäß nimmt die Durchführungs-Öffnung höchstens die Hälfte der Fläche der gebogenen Oberfläche ein, bevorzugt höchstens 35 %. Bevorzugt nimmt die Durchführungs-Öffnung mindestens 20 % der Fläche der gebogenen Oberfläche ein. In einer anderen Ausgestaltung nimmt die Durchführungs-Öffnung höchstens 10 % oder sogar höchstens 5 % der Fläche ein.

Erfindungsgemäß ist eine Durchführungs-Öffnung in das flächige Element und damit in die gebogene Oberfläche des Anschlussstücks eingelassen. Diese Durchführungs-Öffnung nimmt höchstens die Hälfte der Fläche der gebogenen Oberfläche ein, aber nicht die gesamte gebogene Oberfläche. Bevorzugt nimmt die Durchführungs-Öffnung einen geringeren Teil als die Hälfte ein, insbesondere höchstens ein Viertel. Bevorzugt nimmt die Durchführungs-Öffnung mindestens ein Fünftel der Fläche der gebogenen Oberfläche ein.

Weil die Durchführungs-Öffnung erfindungsgemäß höchstens die Hälfte der Fläche einnimmt, ist einerseits der Druckverlust, der beim Einführen des weiteren Geräts entsteht oder entstehen kann, geringer, als wenn die Durchführungs-Öffnung einen größeren Teil der gebogenen Oberfläche oder sogar die gesamte Breite des Anschlussstücks einnehmen würde. Weil der Druckverlust beim Einführen des weiteren Geräts geringer ist, wird insbesondere die Gefahr reduziert, dass aus der Lunge des Patienten in unerwünschter Weise Luft entweicht und die Lunge dadurch in sich zusammenfällt. Andererseits ist die Durchführungs-Öffnung für viele Anwendungen groß genug.

In einer Ausgestaltung ist das Anschlussstück fest mit dem Mittelstück verbunden, beispielsweise stoffschlüssig durch eine Schweißverbindung oder eine Klebeverbindung oder durch eine geeignete formschlüssige Verbindung. In einer anderen Ausgestaltung lässt sich das Anschlussstück lösbar in das Mittelstück einsetzen und wieder aus dem Mittelstück entnehmen. Es ist möglich, dass unterschiedliche Anschlussstücke, auch mit verschiedenen Geometrien und / oder mit verschiedenen Abmessungen und / oder aus verschiedenen Materialien, sich nacheinander in das Mittelstück einsetzen und wieder entnehmen lassen. In vielen Fällen reicht es aus, ein passendes Anschlussstück einzusetzen, um ein weiteres Gerät einzuführen. Nicht erforderlich ist es, unterschiedliche Verbindungseinheiten zu verwenden.

Erfindungsgemäß lässt sich das Anschlussstück in das Mittelstück einsetzen. In einer Ausgestaltung rastet das Anschlussstück ein, wenn es beim Einsetzen eine Position erreicht, in welche die gebogene Oberfläche bündig mit der Innenwand des gebogenen Röhrensegments des Mittelstücks abschließt. Das Einrasten wird beispielsweise durch einen umlaufenden Vorsprung am Anschlussstück oder am Mittelstück realisiert. Diese Ausgestaltung ermöglicht es, dass zwischen dem Anschlussstück und dem Mittelstück einen Spalt auftritt, was das Einsetzen des Anschlussstücks erleichtert. Trotzdem ist das Anschlussstück nach dem Einsetzen fluiddicht mit dem Mittelstück verbunden. Es ist möglich, dass nach dem Einrasten das Anschlussstück fest mit dem Mittelstück verbunden ist. Möglich ist auch, dass das eingerastete Anschlussstück sich wieder aus dem Mittelstück herausziehen lässt.

Möglich ist auch, dass das Anschlussstück kraftschlüssig im Mittelstück gehalten wird, z.B. durch eine Schnapphalterung oder durch einen Wulst.

In einer Ausgestaltung ist das Anschlussstück aus einem Anschlussstück-Material hergestellt. Das Mittelstück und die übrigen Bestandteile der erfindungsgemäßen Verbindungseinheit sind aus mindestens einem sonstigen Material hergestellt. Das Anschlussstück-Material weist bevorzugt eine höhere Elastizität auf als das oder jedes Material des Mittelstücks, bevorzugt eine höhere Elastizität als das oder jedes Material der Verbindungseinheit außer dem Anschlussstück. Diese Ausgestaltung mit dem flexiblen und bevorzugt biegsamen Anschlussstück erleichtert es, das Anschlussstück in die Aufnahme-Öffnung des Mittelstücks einzusetzen, und zwar so, dass das Anschlussstück die Aufnahme-Öffnung fluiddicht ausfüllt. Dank der höheren Elastizität wird in vielen Fällen kein Fügeverfahren benötigt, um das Anschlussstück fluiddicht mit dem Mittelstück zu verbinden. Insbesondere ist in vielen Fällen keine stoffschlüssige Verbindung erforderlich. Lediglich das Anschlussstück wird eingesetzt.

Bevorzugt umfasst die Verbindungseinheit eine Kappe. Mithilfe dieser Kappe lässt sich das gerade Röhrensegment des Anschlussstücks reversibel verschließen, d.h. die Kappe lässt sich wieder abnehmen. Dank dieser Kappe ist das gerade Röhrensegment gegen die Umgebung verschlossen und dadurch gegen das unerwünschte Eindringen eines Fluids oder von Partikeln geschützt, solange kein weiteres Gerät eingeführt ist. Die Kappe braucht lediglich zu dem Zweck abgenommen zu werden, das weitere Gerät einzuführen, und verhindert das Austreten einer großen Menge von Fluid aus eine Fluidverbindung zwischen dem medizinischen Gerät und dem Patienten. Nachdem das weitere Gerät wieder entnommen ist, lässt sich die Kappe wieder aufsetzen.

In einer Ausgestaltung ist die Durchführungs-Öffnung durch ein biegsames Element verschlossen. Dieses biegsame Element wird geöffnet, wenn das weitere Gerät eingeführt wird. Insbesondere bei dieser Ausgestaltung braucht die Kappe nicht notwendigerweise das gerade Röhrensegment fluiddicht zu verschließen.

Besonders bevorzugt wird die Kappe durch eine geeignete Halterung an dem Anschlussstück oder an einem sonstigen Bestandteil der erfindungsgemäßen Verbindungseinheit gehalten. Dies verhindert, dass die Kappe abfällt und verloren geht.

Das gerade Röhrensegment und das Mittelstück stellen erfindungsgemäß zusammen eine gerade Röhre bereit, durch welche hindurch sich das weitere Gerät einführen lässt. Bevorzugt setzt sich diese gerade Röhre gerade, d.h. ohne Richtungsänderung, im patientenseitigen Verbindungsstück und in einer angeschlossenen patientenseitigen Fluidführungseinheit fort. Dank dieser Ausgestaltung kann das weitere Gerät ein starres längliches, z.B. stabförmiges Bauteil umfassen, welches sich ohne Verkanten durch die patientenseitige Fluidführungseinheit hindurch verschieben lässt. Die Gefahr wird reduziert, dass das weitere Gerät verkantet oder dass eine Wand der Verbindungseinheit von innen beschädigt oder die Verbindungseinheit selber beschädigt wird.

Um ein weiteres Gerät einzuführen, muss das weitere Gerät durch das flächige Element mit der gebogenen Oberfläche des Anschlussstücks geführt werden. Um dies zu ermöglichen, weist das flächige Element erfindungsgemäß eine Durchführungs-Öffnung auf. In einer bevorzugten Ausgestaltung wird diese Durchführungs-Öffnung wenigstens teilweise von zwei einander gegenüberliegenden Dichtlippen begrenzt. In einer anderen Ausgestaltung wird die Durchführungs-Öffnung von einer umlaufenden Dichtlippe bereitgestellt.

Beim Einführen des weiteren Geräts lässt sich die oder jede Dichtlippe zur Seite schieben, sodass die Durchführungs-Öffnung vergrößert wird. Ein ausreichend starres weiteres Gerät schiebt beim Einführen die Dichtlippen von allein zur Seite. Solange kein weiteres Gerät eingeführt ist, weist die Durchführungs-Öffnung eine geringere Querschnittsfläche auf, verglichen mit den Zustand bei eingeführtem weiteren Gerät. Die Dichtlippe reduziert die Menge von Fluid, welches durch die Durchführungs-Öffnung hindurch entweicht, verglichen mit einem starren Rand um die Durchführungs-Öffnung. Die Durchführungs-Öffnung kann größer ausgestaltet sein, ohne dass bei eingesetztem Gerät größere Leckageverluste auftreten. Die oder jede Dichtlippe ist bevorzugt aus einem elastischen Material hergestellt.

Erfindungsgemäß stellt die Verbindungseinheit eine gekrümmte Röhre bereit. Diese gekrümmte Röhre ändert die Fließrichtung eines Fluids, welches durch die Verbindungseinheit fließt. Bevorzugt liegt der Winkel, um den die Fließrichtung geändert wird, zwischen 35° und 90°, besonders bevorzugt zwischen 50° und 80° und insbesondere zwischen 60° und 70°.

In einer Ausgestaltung wird die Krümmung der bereitgestellten gekrümmten Röhre nur durch das gekrümmte Röhrensegment bewirkt. Die beiden hohlen Verbindungsstücke stellen in dieser Ausgestaltung zwei gerade Röhren bereit.

Das gekrümmte Röhrensegment des Mittelstücks gehört zur bereitgestellten gekrümmten Röhre und weist eine Wand auf. Bevorzugt umfasst diese Wand ein gebogenes inneres Wandsegment und ein gebogenes äußeres Wandsegment. Die Begriffe "innen" und "außen" beziehen sich auf die Krümmungsrichtung des gekrümmten Röhrensegments. Die beiden Wandsegmente können fest miteinander verbunden und insbesondere einstückig ausgebildet sein. Bevorzugt bildet die gebogene Oberfläche des Anschlussstücks einen Teil des äußeren Wandsegments oder das gesamte äußere Wandsegment. Die Durchführungs-Öffnung ist also in das äußere Wandsegment eingelassen. In die Fließrichtung gesehen kann die gebogene Oberfläche die gesamte Länge des äußeren Wandsegments einnehmen.

Die Ausgestaltung mit Dichtlippen und die Ausgestaltung mit der Kappe lassen sich miteinander kombinieren. Dank der Dichtlippen ist es nicht erforderlich, dass die Kappe das Anschlussstück vollständig fluiddicht verschließt, auch nicht bei einem Überdruck innerhalb der Verbindungseinheit. Ausreichend ist, dass die aufgesetzte Kappe den Volumenfluss ausreichend reduziert, verglichen mit einer Situation, bei der die Kappe nicht aufgesetzt ist.

Erfindungsgemäß ist das Anschlussstück so in das Mittelstück eingesetzt oder lässt sich so einsetzen, dass die gebogene Oberfläche des Anschlussstücks einen Teil der Wand des gebogenen Röhrensegments bildet. Diese Ausgestaltung erleichtert in vielen Fällen die Herstellung der erfindungsgemäßen Verbindungseinheit mit dem gebogenen Röhrensegment. Es ist möglich, das Anschlussstück und die sonstigen Bestandteile der Verbindungseinheit getrennt voneinander herzustellen, wobei das Mittelstück zwischen den beiden Verbindungsstücken eine ausreichend große Öffnung aufweist. Später wird das Anschlussstück in diese Aufnahme-Öffnung eingesetzt.

Bevorzugt umfasst das Mittelstück einen Stutzen. Dieser Stutzen umgibt die Aufnahme-Öffnung des Mittelstücks. Das Anschlussstück lässt sich in den Stutzen einsetzen oder ist in den Stutzen eingesetzt. Die Ausgestaltung mit dem Stutzen erleichtert es, das Anschlussstück in das Mittelstück einzusetzen. Falls zum Herstellen der erfindungsgemäßen Verbindungseinheit ein Roboter verwendet wird, vermag dieser Stutzen einen Greifarm oder ein anderes Teil dieses Roboters zu führen.

Erfindungsgemäß umfasst die Verbindungseinheit ein Mittelstück und zwei Verbindungsstücke. Das Mittelstück ist zwischen den beiden Verbindungsstücken angeordnet. Die beiden Enden des Mittelstücks sind mit jeweils einem Verbindungsstück verbunden. Bevorzugt ist das Mittelstück mit jedem Verbindungsstück jeweils drehbar verbunden, und zwar drehbar um eine Drehachse, die parallel zur Mittelachse des jeweiligen Verbindungsstücks verläuft. Die beiden Drehachsen stehen senkrecht oder schräg aufeinander.

Diese drehbare Ausgestaltung reduziert die mechanische Spannung, der ein Fluidführungssystem einer Fluidverbindung zwischen dem medizinischen Gerät und der patientenseitigen Kopplungseinheit ausgesetzt ist, wobei dieses Fluidführungssystem die erfindungsgemäße Verbindungseinheit, eine mit dem patientenseitigen Verbindungsstück verbundene patientenseitige Fluidführungseinheit und eine mit dem geräteseitigen Verbindungsstück verbundene geräteseitige Fluidführungseinheit umfasst. Mindestens zwei drehbare Verbindungen mit unterschiedlichen Drehachsen erleichtern es, dass das Fluidführungssystem sich von alleine so ausrichtet, dass die geringste mögliche mechanische Spannung auftritt. Dies reduziert die Gefahr, dass die Fluidverbindung durch einen Knick unterbrochen wird und dadurch der Fluss von Fluid beeinträchtigt wird, oder dass durch eine hohe Biegespannung eine Beschädigung, insbesondere ein Leck, auftritt.

Die Erfindung betrifft weiterhin ein Fluidführungs-Bauteil, welches
- ein Fluidführungssystem umfassend eine geräteseitige Fluidführungseinheit und
- eine erfindungsgemäße Verbindungseinheit
umfasst. Das geräteseitige Verbindungsstück der Verbindungseinheit ist fluiddicht mit der geräteseitigen Fluidführungseinheit verbunden oder verbindbar.

Die Erfindung betrifft weiterhin eine Fluidführungs-Anordnung, welche ein Fluidführungs-Bauteil und eine patientenseitige Fluidführungseinheit umfasst. Das Fluidführungs-Bauteil ist so ausgestaltet wie gerade beschrieben. Das patientenseitige Verbindungsstück der Verbindungseinheit ist fluiddicht mit der patientenseitigen Fluidführungseinheit verbunden oder lässt sich mit dieser verbinden. Eine patientenseitige Fluidführungseinheit ist fluiddicht mit einer patientenseitigen Kopplungseinheit verbunden oder lässt sich mit dieser verbinden.

Weiterhin betrifft die Erfindung ein Fluidführungssystem. Dieses Fluidführungssystem umfasst eine Fluidführungs-Anordnung, die so ausgestaltet ist wie gerade beschrieben, und eine patientenseitige Kopplungseinheit. Die patientenseitige Fluidführungseinheit ist fluiddicht mit einer patientenseitigen Kopplungseinheit verbunden oder lässt sich mit einer solchen verbinden.

Die Erfindung betrifft weiterhin eine medizinische Anordnung, welche für eine medizinischen Behandlung eines Patienten ausgestaltet ist. Diese medizinische Anordnung umfasst
- ein medizinisches Gerät, das ein Beatmungsgerät ist, welches als ein Anästhesiegerät ausgestaltet sein kann,
- eine geräteseitige Fluidführungseinheit,
- eine patientenseitigen Fluidführungseinheit,
- eine patientenseitige Kopplungseinheit, die sich lösbar mit einem Patienten verbinden lässt und sich insbesondere auf das Gesicht legen oder in den Körper des Patienten einführen lässt, und
- eine erfindungsgemäße Verbindungseinheit.

Das medizinische Gerät ist fluiddicht mit der geräteseitigen Fluidführungseinheit verbunden oder mit ihr verbindbar. Die geräteseitige Fluidführungseinheit ist fluiddicht mit dem geräteseitigen Verbindungsstück der Verbindungseinheit verbunden oder mit ihr verbindbar. Das patientenseitige Verbindungsstück der Verbindungseinheit ist fluiddicht mit der patientenseitigen Fluidführungseinheit verbunden oder verbindbar. Die patientenseitige Fluidführungseinheit ist fluiddicht mit der patientenseitigen Kopplungseinheit verbunden oder verbindbar.

Die Erfindung betrifft auch eine Verwendung einer erfindungsgemäßen Verbindungseinheit oder eines erfindungsgemäßen Fluidführungs-Bauteils oder einer erfindungsgemäßen Fluidführungs-Anordnung oder eines erfindungsgemäßen Fluidführungssystems zum Herstellen einer Fluidverbindung zwischen einer patientenseitigen Kopplungseinheit und einem medizinischen Gerät, insbesondere einem Beatmungsgerät.

Die Erfindung betrifft auch ein Verfahren zur Herstellung einer erfindungsgemäßen Verbindungseinheit umfassend die folgenden Schritte:
- Das Anschlussstück wird hergestellt.
- Die beiden Verbindungsstücke werden hergestellt.
- Das Mittelstück wird hergestellt, und zwar so, dass sich in der Wand des Mittelstücks die Aufnahme-Öffnung befindet.
- Das Anschlussstück wird in die Aufnahme-Öffnung eingesetzt. Das Einsetzen wird dergestalt durchgeführt, dass die gebogene Oberfläche des Anschlussstücks die Aufnahme-Öffnung verschließt.
- Das Mittelstück wird mit den beiden Verbindungsstücken fluiddicht verbunden.

Dieses Herstellungsverfahren ermöglicht es, das Anschlussstück getrennt von den übrigen Bestandteilen der Verbindungseinheit herzustellen. In vielen Fällen ist es möglich, jedes Bestandteil der Verbindungseinheit ohne Hinterschneidungen herzustellen.

Es ist möglich, jeweils eine Menge von gleichartigen patientenseitigen Verbindungsstücken, geräteseitigen Verbindungsstücken und Mittelstücken sowie mindestens zwei Mengen von zwei unterschiedlichen Anschlussstücken herzustellen, wobei diese Anschlussstücke sich in ihrer Geometrie und / oder in mindestens einer Abmessung unterscheiden. Beide unterschiedliche Anschlussstücke lassen sich in das Mittelstück einsetzen, nämlich wahlweise das eine oder das andere Anschlussstück. Diese Ausgestaltung erleichtert es, Verbindungseinheiten für unterschiedliche einführbare weitere Geräte herzustellen und dennoch beim Herstellen der Verbindungsstücke und Mittelstücke die Vorteile einer Fertigung mit großer Stückzahl zu nutzen. Die Verbindungsstücke und Mittelstücke lassen sich jeweils mit einer größeren Stückzahl herstellen, die Mengen von Anschlussstücken mit jeweils einer kleineren Stückzahl.

In einer bevorzugten Ausgestaltung wird zuerst das Anschlussstück in das Mittelstück eingesetzt. Anschließend werden die beiden Verbindungsstücke mit dem Mittelstück fluiddicht verbunden. Diese Ausgestaltung erleichtert es, eine optionale Halteschlaufe für eine optionale Kappe anzubringen. In einer anderen Ausgestaltung werden zuerst die beiden Verbindungsstücke mit dem Mittelstück zu einem Bauteil verbunden, und anschließend wird das Anschlussstück eingesetzt. Möglich ist auch, das Mittelstück und die beiden Verbindungsstücke als ein Bauteil herzustellen und das Anschlussstück in dieses eine Bauteil einzusetzen.

Bevorzugt werden die beiden Verbindungsstücke und das Mittelstück aus dem gleichen Material hergestellt. Bevorzugt werden die beiden Verbindungsstücke und das Mittelstück durch ein Spritzgießverfahren hergestellt.

Außerdem betrifft die Erfindung ein Computerprogramm, welches auf einen 3D-Drucker ausgeführt werden kann. Eine Rechnereinheit, welches das Computerprogramm auszuführen vermag, kann Bestandteil des 3D-Druckers sein oder räumlich vom 3D-Drucker entfernt sein. Wird das Computerprogramm auf der Recheneinheit und damit auf dem 3D-Drucker ausgeführt, so steuert der das Computerprogramm den 3D-Drucker an. Der vom Computerprogramm angesteuerte 3D-Drucker druckt dann mindestens die beiden Verbindungsstücke, das Anschlussstück und das Mittelstück einer erfindungsgemäßen Verbindungseinheit Verbindungseinheit aus. Darüber hinaus betrifft die Erfindung einen 3D-Drucker, der dazu ausgestaltet ist, mindestens die beiden Verbindungsstücke, das Anschlussstück und das Mittelstück einer erfindungsgemäßen Verbindungseinheit auszudrucken, wobei der 3D-Drucker einen Speicher mit einem erfindungsgemäßen Computerprogramm umfasst.

Im Folgenden wird die Erfindung anhand eines Ausführungsbeispiels beschrieben. Hierbei zeigen
- Figur 1: ein Beatmungsgerät, das mittels eines Fluidführungssystems mit einer patientenseitigen Kopplungseinheit im Körper eines zu beatmenden Patienten verbunden ist;
- Figur 2, Figur 3: die erfindungsgemäße Verbindungseinheit in zwei Querschnittsdarstellungen;
- Figur 4, Figur 5: die erfindungsgemäße Verbindungseinheit in zwei perspektivischen Darstellungen aus zwei verschiedenen Betrachtungsrichtungen;
- Figur 6: die erfindungsgemäße Verbindungseinheit in einer Betrachtungsrichtung parallel zur patientenseitigen Fluidführungseinheit;
- Figur 7, Figur 8: das Anschlussstück in zwei perspektivischen Darstellungen.

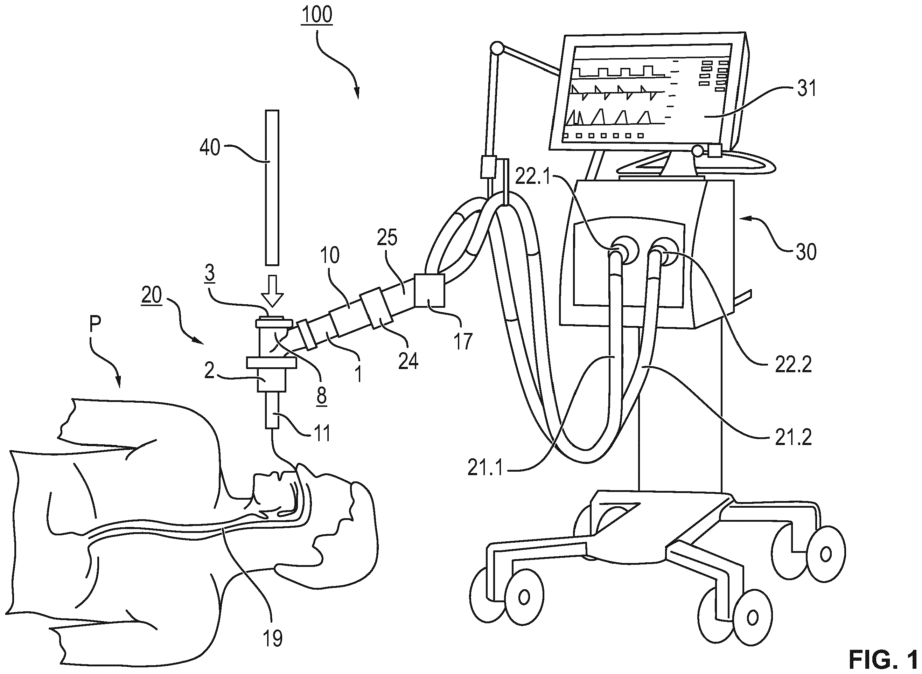

Figur 1 zeigt ein Beatmungsgerät 30, welches einen Patienten P künstlich zu beatmen vermag. Dieses Beatmungsgerät 30 kann auch als ein Anästhesiegerät ausgestaltet sein, welches dem Patienten mindestens ein Narkosemittel zuführt. Figur 1 zeigt weiterhin den künstlich beatmeten Patienten P.

Auf einem Anzeigegerät 31 des Beatmungsgeräts 30 werden die zeitlichen Verläufe von verschiedenen Vitalparametern des Patienten P angezeigt. Das Beatmungsgerät 30 steht in einer fluiddichten Fluidverbindung mit dem Patienten P, sodass Fluid vom Beatmungsgerät 30 zum Patienten P fließen kann. Optional wird ein Beatmungskreislauf realisiert, in dem Fluid sowohl vom Beatmungsgerät 30 zum Patienten P als auch umgekehrt vom Patienten P zum Beatmungsgerät 30 fließen kann.

Diese Fluidverbindung wird durch ein Fluidführungssystem 100 realisiert, welches im gezeigten Ausführungsbeispiel folgende Bestandteile umfasst:
- eine patientenseitige Kopplungseinheit 19 in Form eines Messschlauchs, der in die Speiseröhre des Patienten P eingeführt ist und beispielsweise bis zum Magen reicht, oder in Form eines Beatmungstubus, der in die Luftröhre des Patienten P eingeführt ist und bis zur Lunge reicht,
- eine patientenseitige Fluidführungseinheit in Form eines im gezeigten Beispiel röhrenförmigen Konnektors 11, welcher fluiddicht mit der patientenseitigen Kopplungseinheit 19 verbunden ist,
- ein röhrenförmiger Konnektor 10, der zu einer geräteseitigen Fluidführungseinheit gehört,
- zwei parallele Schläuche 21.1 und 21.2 für die Inspiration bzw. für die Exspiration,
- zwei Verbindungselemente 22.1 und 22.2, welche die beiden geräteseitigen Schläuche 21.1 bzw. 21.2 drehbar mit dem Beatmungsgerät 30 verbinden,
- ein Y-Stück 17, welches die beiden Schläuche 21.1 und 21.2 mit dem Konnektor 10 verbindet,
- eine Filtereinheit und
- eine erfindungsgemäße Verbindungseinheit 20.

Die Filtereinheit umfasst ein Filterelement 24 sowie zwei Fluidführungseinheiten 10, 25 in Form von zwei röhrenförmigen Konnektoren, zwischen denen sich das Filterelement 24 befindet. Die erfindungsgemäße Verbindungseinheit 20 ist mit beiden Konnektoren 10 und 11 fluiddicht und drehbar verbunden. Das Y-Stück 17 verbindet die beiden geräteseitigen Schläuche 21.1 und 21.2 mit dem Konnektor 25 und dadurch indirekt auch mit dem Konnektor 10.

Die beiden Schläuche 21.1 und 21.2, die Konnektoren 10 und 25 sowie das Y-Stück 17 gehören zu der geräteseitigen Fluidführungseinheit des Ausführungsbeispiels. Diese geräteseitige Fluidführungseinheit 10, 17, 21.1, 21.2, 25 sowie die Verbindungseinheit 20 bilden zusammen ein Fluidführungs-Bauteil. Das Fluidführungs-Bauteil 10, 17, 20, 21.1, 21.2, 25 und die patientenseitige Fluidführungseinheit 11 bilden zusammen eine Fluidführungs-Anordnung. Die Fluidführungs-Anordnung 10, 11, 17, 20, 21.1, 21.2, 25 und die patientenseitige Kopplungseinheit 19 bilden zusammen ein Fluidführungssystem.

Anstelle eines Beatmungstubus oder Messschlauchs 19 im Körper des Patienten P kann auch eine Atemmaske auf dem Gesicht des Patienten P zum Fluidführungssystem 100 gehören. Möglich ist, dass das patientenseitige Verbindungsstück 1 der Verbindungseinheit 20 direkt mit einem Konnektor dieser Atemmaske verbunden ist.

Die Fluidführungseinheiten 10, 11, 17, 21.1, 21.2 und 25 vermögen ein Fluid, insbesondere Atemluft oder ein Trägergas mit mindestens einem Anästhesiemittel, zu führen. Das Beatmungsgerät 30 bewirkt, dass das Fluid zum Patienten P fließt. Ein nicht gezeigter Volumenfluss-Sensor ("flow sensor") misst den Volumenfluss im Fluidführungssystem 100, also das fließende Volumen pro Zeiteinheit. Die Werte vom Volumenfluss-Sensor werden z.B. dafür verwendet, um automatisch ein zeitlich veränderliches Maß für die eigene Atmungsaktivität (spontane Atmung) des Patienten P herzuleiten. Dieses Maß lässt sich z.B. dafür verwenden, um automatisch das Beatmungsgerät 30 oder die Zugabe eines Anästhesiemittels zu regeln.

Die erfindungsgemäße Verbindungseinheit 20 ermöglicht den beiden sich anschließenden Fluidführungseinheiten (Konnektoren) 10 und 11 mehrere Freiheitsgrade bei den möglichen Relativbewegungen: Der Konnektor 11 kann sich relativ zur Verbindungseinheit 20 um seine eigene Längsachse drehen. Der Konnektor 10 kann sich ebenfalls relativ zur Verbindungseinheit 20 um seine eigene Längsachse drehen. Wie diese drehbare Verbindung um zwei Längsachsen realisiert wird, die schräg aufeinander stehen, wird weiter unten beschrieben. Außerdem können die beiden Schläuche 21.1 und 21.2 sich in den Verbindungselementen 22.1 und 22.2 relativ zum Beatmungsgerät 30 um ihre jeweilige Längsachse drehen.

Insbesondere dank dieser Eigenschaften der Verbindungen werden die Schläuche 21.1, 21.2 und die Konnektoren 25, 10, 11 des Fluidführungssystems 100 weitgehend frei von mechanischen Spannungen und Verbiegungen und Verdrehungen am Beatmungsgerät 30 gehalten.

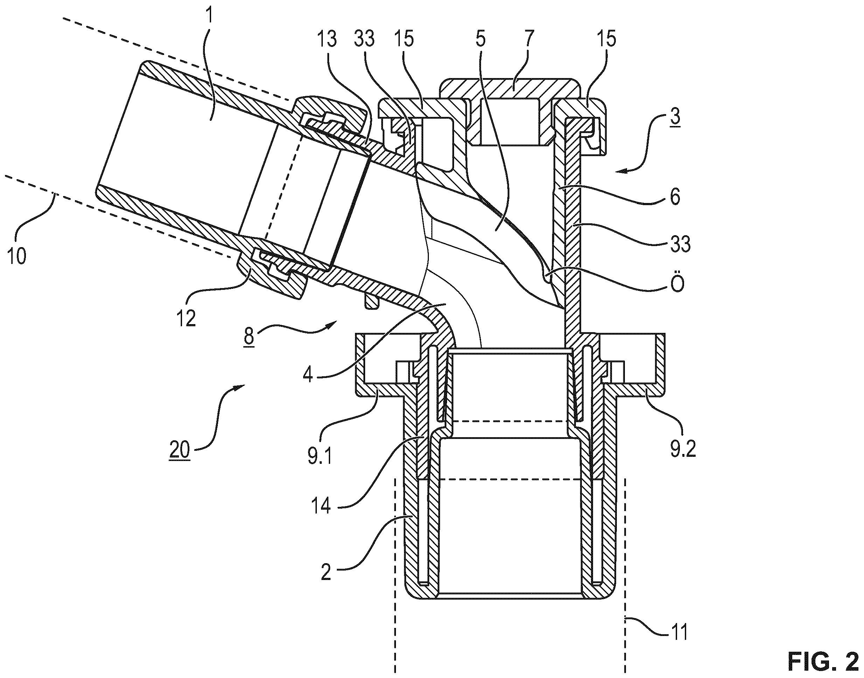

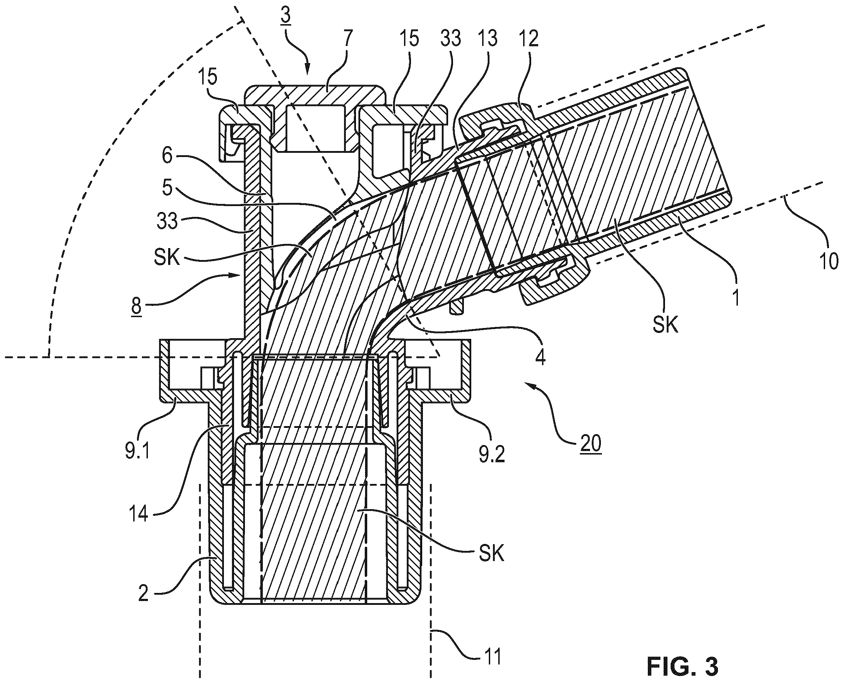

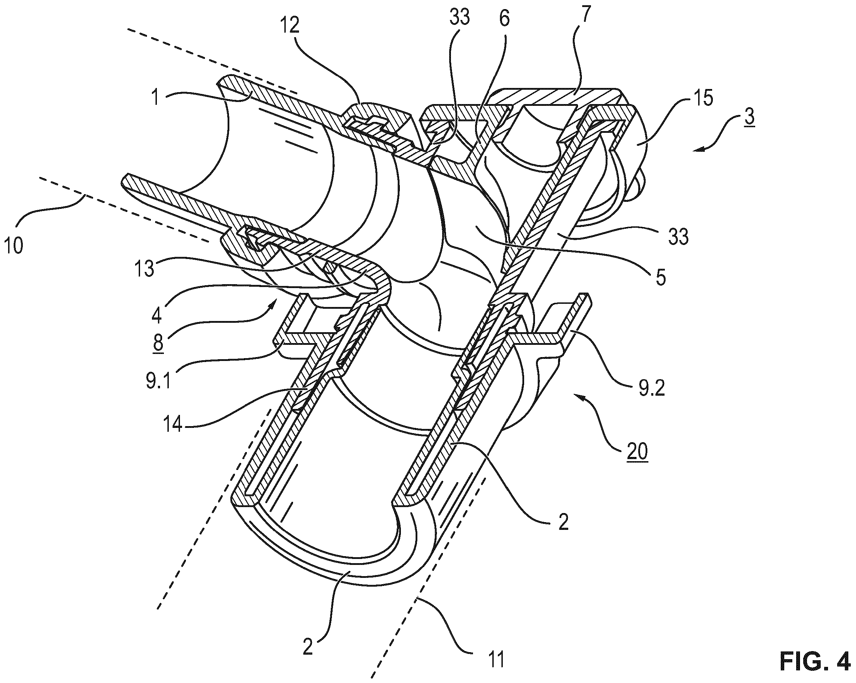

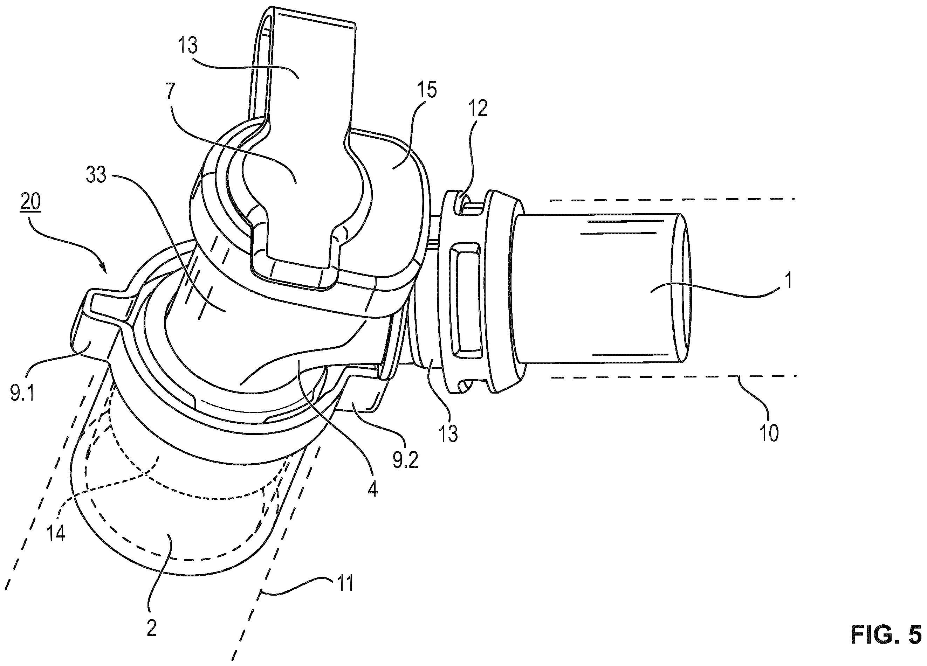

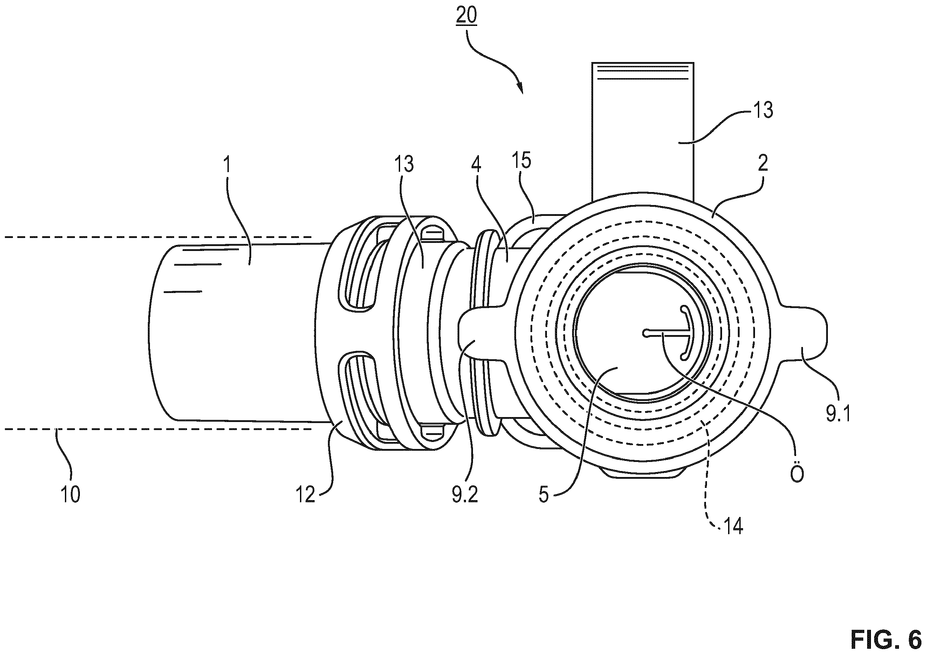

Figur 2 und Figur 3 zeigen die erfindungsgemäße Verbindungseinheit 20 in zwei Querschnittsdarstellungen, Figur 4 und Figur 5 in zwei verschiedenen perspektivischen Darstellungen, Figur 6 in einer Betrachtungsrichtung parallel zur patientenseitigen Fluidführungseinheit (Konnektor) 11. Außerdem werden die patientenseitige Fluidführungseinheit (Konnektor 11) und die geräteseitige Fluidführungseinheit (Konnektor 10) durch gestrichelte Linien angedeutet.

Die Verbindungseinheit 20 des Ausführungsbeispiels umfasst folgende Bestandteile:
- ein Mittelstück ("body") 8,
- ein geräteseitiges Verbindungsstück 1,
- ein patientenseitiges Verbindungsstück 2 und
- ein Anschlussstück 3.

Das Mittelstück 8 umfasst
- ein geräteseitiges gerades Röhrensegment 13,
- ein gekrümmtes Röhrensegment 4,
- einen geraden Stutzen 33 und
- ein patientenseitiges gerades Röhrensegment 14.

Die drei Röhrensegmente 13, 4 und 14 sowie der Stutzen 33 sind fest und fluiddicht miteinander verbunden und gehen kontinuierlich ineinander über. Insbesondere stoßen zwei benachbarte Röhrensegmente 13 und 4 sowie 4 und 14 nicht in einer innenliegenden Kante aneinander an. Zwischen dem geräteseitigen geraden Röhrensegment 13 und dem Stutzen 33 kann eine Kante auftreten.

Die beiden Verbindungsstücke 1 und 2, die drei Röhrensegmente 13, 4 und 14 sowie eine weiter unten beschriebene gebogene Oberfläche eines flächigen Elements 5 stellen gemeinsam eine teils geraden und teils gebogene Röhre (Strömungskanal SK) bereit, wobei ein Fluid durch den Strömungskanal SK in beide Richtungen fließen kann und wobei der Strömungskanal SK im Ausführungsbeispiel durchgehend die gleiche Querschnittsfläche für ein Fluid aufweist. Möglich ist auch, dass der Strömungskanal SK sich in eine Fließrichtung verjüngt.

Im gekrümmten Röhrensegment 4 verändert sich die Richtung dieses Strömungskanals SK kontinuierlich. Das gebogene Mittelstück 8 lenkt einen Fluidstrom, der durch den Strömungskanal SK fließt, um einen Winkel um, der bevorzugt zwischen 35° und 90° liegt, besonders bevorzugt zwischen 50° und 80°, insbesondere zwischen 60° und 70°. Der vom Stutzen 33 bereitgestellte und bevorzugt gerade Kanal mündet in diesen Strömungskanal SK ein.

Das Merkmal, dass der Strömungskanal SK im gekrümmten Röhrensegment 4 seine Richtung kontinuierlich ändert, tritt bevorzugt bei jeder Position eines Verbindungsstücks 1 oder 2 relativ zum Mittelstück 8 auf, also unabhängig von der Rotationsposition eines Verbindungsstücks 1 oder 2. Dieses Merkmal der kontinuierlichen Richtungsänderung verringert die Gefahr, dass Verwirbelungen im gekrümmten Teil des Strömungskanals SK auftreten. Dieses Merkmal wird insbesondere dadurch erzielt, dass die Innenwände der drei Röhrensegmente 13, 4 und 14 sowie die gebogene Oberfläche des flächigen Elements 5 keine Kanten aufweisen, auch nicht an einem Übergang. Der bereitgestellte Strömungskanal SK weist daher ebenfalls keine Kanten auf. Eine weitere Wirkung, die durch das gewollte Fehlen von Kanten erzielt wird, ist die folgende: Die Strömung durch den Strömungskanal SK variiert geringer über der Position und / oder über der Zeit, und die Werte, die der nicht gezeigte Volumenfluss-Sensor liefert, streuen weniger und sind zuverlässiger als beim Vorhandensein einer Kante.

Das geräteseitige Verbindungsstück 1 ist drehbar und fluiddicht mit dem geräteseitigen geraden Röhrensegment 13 des Mittelstücks 8 verbunden und umfasst einen Konus 12. Das patientenseitige Verbindungsstück 2 ist drehbar und fluiddicht mit dem patientenseitigen geraden Röhrensegment 14 des Mittelstücks 8 verbunden und umfasst in einer Ausführungsform zwei Griffelemente 9.1 und 9.2. Möglich ist auch, nur ein Griffelement oder mehr als zwei Griffelemente oder auch überhaupt kein Griffelement vorzusehen. Die beiden Verbindungsstücke 1 und 2 umschließen jeweils einen Teil eines geraden Röhrensegments 13 bzw. 14.

Die patientenseitige Fluidführungseinheit, im Ausführungsbeispiel der Konnektor 11 für den Beatmungstubus oder den Messschlauch 19 oder für eine Atemmaske des Fluidführungssystems 100, lässt sich fluiddicht und drehfest mit dem patientenseitigen Verbindungsstück 2 verbinden. Bevorzugt wird der Konnektor 11 vom patientenseitigen Verbindungsstück 2 umschlossen, d.h. ein geräteseitiges Ende des Konnektors 11 befindet sich innerhalb des patientenseitigen Verbindungsstücks 2. Falls als patientenseitige Fluidführungseinheit eine Atemmaske verwendet wird, so umschließt dessen Konnektor bevorzugt das patientenseitige Verbindungsstück 2. Bevorzugt ist das patientenseitige Verbindungsstück 2 als ein M22/F15-Doppelkonus nach ISO 5356 - 1 ausgestaltet, der eine Steigung von 1:40 aufweist.

Die geräteseitige Fluidführungseinheit, im Ausführungsbeispiel der Konnektor 10, lässt sich fluiddicht und drehfest mit dem geräteseitigen Verbindungsstück 1 verbinden. Bevorzugt umschließt das patientenseitige Ende des Konnektors 10 das geräteseitige Verbindungsstück 1. Das geräteseitige Verbindungsstück 1 ist bevorzugt als ein M15-Konus nach ISO 5356 - 1 ausgestaltet.

Das gekrümmte Röhrensegment 4 des Mittelstücks 8 weist in seiner - gesehen in die Krümmungsrichtung - äußeren Wand eine Aufnahme-Öffnung auf. In einer Ausgestaltung erstreckt sich diese Aufnahme-Öffnung über die gesamte Abmessung - gesehen in Strömungsrichtung - der äußeren Wand. Das zum Mittelstück 8 hin zeigende Ende des geraden Stutzens 33 umgibt diese Aufnahme-Öffnung.

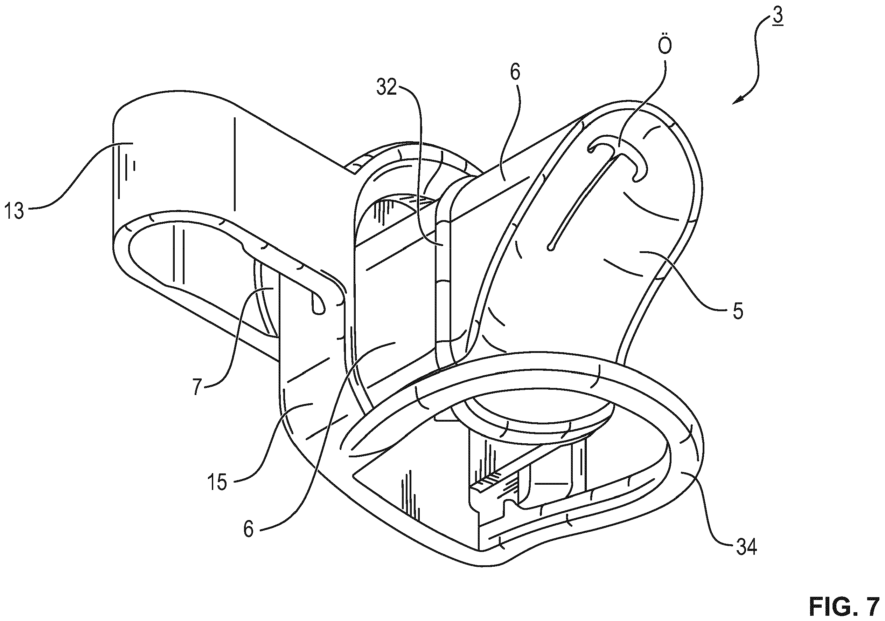

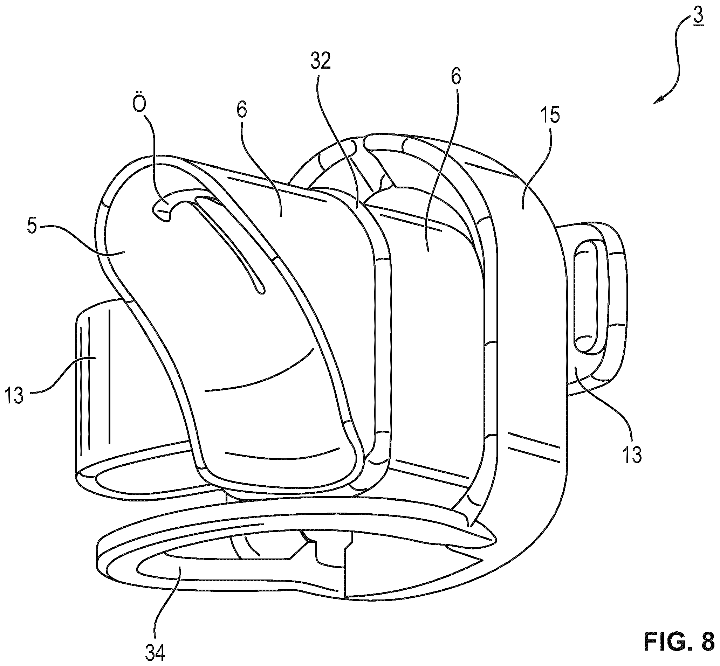

Figur 7 und Figur 8 zeigen in zwei perspektivischen Darstellungen aus zwei unterschiedlichen Betrachtungsrichtungen das Anschlussstück 3. Das Anschlussstück 3 umfasst
- ein gerades Röhrensegment 6 mit einem umlaufenden Vorsprung 32,
- eine optionale Krempe 15 an dem vom Mittelstück 8 entfernten Ende des geraden Röhrensegments 6,
- ein flächiges Element 5 mit einer gebogenen Oberfläche 5 an dem anderen Ende des geraden Röhrensegments 6, wobei in das flächige Element 5 eine Durchführungs-Öffnung Ö eingelassen ist,
- eine optionale Schlaufe 34 zum Halten des Anschlussstücks 3,
- eine optionale Kappe 7 und
- eine optionale Halterung 13 für die Kappe 7.

Die Kappe 7 lässt sich in eine Öffnung in der Krempe 15 einsetzen und wieder abnehmen. In Figur 7 und Figur 8 ist die Kappe 7 eingesetzt. Die eingesetzte Kappe 7 verschließt die Krempe 15. Die Halterung 13 hält die Kappe 7 an der Krempe 15 und verhindert, dass die Kappe 7 sich von selbst löst und abfällt, z.B. aufgrund von Vibrationen, oder wenn sie nicht aufgesetzt ist. Die aufgesetzte Kappe 7 verhindert, dass Fluid oder Partikel von außen durch das gerade Röhrensegment 6 hindurch in den Strömungskanal SK fließen oder umgekehrt Fluid aus dem Strömungskanal SK durch das gerade Röhrensegment 6 hindurch nach außen austritt. Das Anschlussstück 3 lässt sich an der Schlaufe 34 anfassen.

Bevorzugt lässt sich das gesamte Anschlussstück 3 in den geraden Stutzen 33 einsetzen. Das flächige Element 5 füllt dann die Aufnahme-Öffnung - bis auf die weiter unten beschriebene Durchführungs-Öffnung Ö - vollständig und fluiddicht aus. Das gerade Röhrensegment 6 verbindet diese Aufnahme-Öffnung mit der Krempe 15. Der gerade Stutzen 33 des Mittelstücks 8 umgibt das gerade Röhrensegment 6 des Anschlussstücks 3. Diese Ausgestaltung ermöglicht es, das Anschlussstück 3 getrennt von dem Rest der Verbindungseinheit 20 herzustellen, und erleichtert es weiterhin, das Anschlussstück 3 aus einem anderen Material als den Rest der Verbindungseinheit 20 herzustellen. Bevorzugt stimmt das Innenprofil des geraden Stutzens 33 mit dem Außenprofil des geraden Röhrensegments 6 überein. Die beiden Profile sind bevorzugt so ausgestaltet, dass das Anschlussstück 3 sich nur in einer bestimmten Position relativ zum geraden Stutzen 33 einsetzen lässt. Die gebogene Oberfläche des flächigen Elements 5 schließt bevorzugt bündig mit der Innenwand des Mittelstücks 8 ab.

In einer Ausgestaltung ist das Anschlussstück 3 durch eine Fügeverbindung fluiddicht mit dem Mittelstück 8 verbunden, beispielsweise durch eine Klebeverbindung oder Schweißverbindung. Bei der Herstellung der Verbindungseinheit 20 wird das Anschlussstück 3 in das Mittelstück 8 eingesetzt und mit diesem verbunden. Dank dieser Fügeverbindung lässt das Anschlussstück 3 sich nicht mehr zerstörungsfrei aus dem Mittelstück 8 herausziehen.

In einer anderen Ausgestaltung lässt sich das Anschlussstück 3 wieder zerstörungsfrei aus dem Mittelstück 8 herausziehen. Bevorzugt halten eine Schnapphalterung oder eine sonstige Rasteinheit das Anschlussstück 3 formschlüssig im Mittelstück.

Bevorzugt ist das gesamte Anschlussstück 3 aus einem elastischen, d.h. reversibel verformbaren Material hergestellt, bevorzugt einstückig oder sogar monolithisch (in einem einzigen Schritt hergestellt). Genauer gesagt: Die Elastizität des Anschlussstücks 3 ist größer als die Elastizität des Mittelstücks 8 und die Elastizität der Verbindungsstücke 1 und 2. Die höhere Elastizität des Anschlussstücks 3 verringert die Gefahr, dass eine Leckage zwischen dem eingesetzten Anschlussstück 3 und dem Mittelstück 8 auftritt. Möglich ist, dass das Anschlussstück 3 beim Einsetzen in den geraden Stutzen 33 des Mittelstücks 8 leicht zusammengedrückt und dadurch reversibel verformt wird und das verformte flächige Element 5 mit der gebogenen Oberfläche 5 sich aufgrund der eigenen Rückstellkraft an den Rand der Aufnahme-Öffnung anschmiegt und dadurch anpasst.

Möglich ist auch, dass zwischen dem geraden Stutzen 33 und dem geraden Röhrensegment 6 des Anschlussstücks 3 ein umlaufender Spalt auftritt. Dieser Spalt erleichtert es, das Anschlussstück 3 in den Stutzen 33 einzusetzen. Der umlaufende Vorsprung 32 drückt von innen gegen den geraden Stutzen 33, verschließt den umlaufenden Spalt und reduziert die Gefahr eines Lecks weiter. In einer Ausgestaltung rastet das gerade Röhrensegment 6 im Stutzen 33 ein.

Das gerade Röhrensegment 6 des Verbindungsstücks 3, das patientenseitige gerade Röhrensegment 14 und das patientenseitige Verbindungsstück 2 stellen zusammen eine gerade durchgehende Röhre bereit, die gerade in die patientenseitige Fluidführungseinheit 11 einmündet und durch das flächige Element 5 unterbrochen und weitgehend verschlossen wird. Das flächige Element 5 nimmt erfindungsgemäß mindestens die Hälfte, bevorzugt mindestens 80 % der Aufnahme-Öffnung ein, wenn kein weiteres Gerät eingesetzt ist. Den Rest der Aufnahme-Öffnung nimmt die Durchführungs-Öffnung Ö ein. In einer Ausgestaltung nimmt das flächige Element 5 mindestens 90 % oder sogar mindestens 95 % der Aufnahmeöffnung ein. In einer Ausgestaltung nimmt die Durchführungs-Öffnung Ö wenigstens 20 % der Aufnahme-Öffnung ein.

Ein intrakorporales Gerät, beispielsweise ein Absaugkatheter oder ein Endoskop oder ein Sensor, lässt sich durch diese gerade Röhre hindurch von außen in die patientenseitige Fluidführungseinheit 11 einführen, ohne die Fluidverbindung zwischen dem Patienten P und dem Beatmungsgerät 30 trennen zu müssen. In Figur 1 werden schematisch ein stabförmiges intrakorporales Gerät 40 sowie durch einen Pfeil die Einführrichtung gezeigt. Mithilfe dieses intrakorporalen Geräts 40 lassen sich beispielsweise Sekrete aus dem Körper des Patienten P absaugen, oder der Patient P oder der Fluidstrom können untersucht werden. Beispielsweise lässt sich eine Messsonde in die Nähe der Lunge des Patienten P verbringen. Die bevorzugt starre patientenseitige Fluidführungseinheit 11 lässt sich dafür verwenden, das intrakorporale Gerät 40 zum Patienten P zu führen, und umgibt das intrakorporale Gerät 40. Auch während das intrakorporale Gerät 40 eingeführt und anschließend verwendet wird, vermag das Beatmungsgerät 30 weiterhin den Patienten P künstlich zu beatmen. Möglich ist auch, durch die gerade Röhre hindurch Medikamente, Kontrastmittel oder sonstige Substanzen in den Körper des Patienten P zu verbringen.

Wie bereits dargelegt, lässt sich bevorzugt das Anschlussstück 3 in die Aufnahme-Öffnung einsetzen. Das Anschlussstück 3 lässt sich getrennt vom Rest der Verbindungseinheit 20 fertigen. Diese Ausgestaltung ermöglicht es, unterschiedliche Verbindungseinheiten für verschiedene Zwecke herzustellen und zu verwenden. Die unterschiedlichen Verbindungseinheiten unterscheiden sich bevorzugt ausschließlich durch verschiedene Anschlussstücke 3, beispielsweise verschiedene Durchführungs-Öffnungen Ö und / oder unterschiedlich großen Stutzen 33, während die Verbindungseinheiten ansonsten gleichartig aufgebaut sind. Diese Ausgestaltung reduziert die Varianz und ermöglicht es, den Rest der Verbindungseinheit 20 in einer größeren Stückzahl herzustellen und nur das Anschlussstück 3 in Varianten für die unterschiedlichen Verwendungen und jeweils in kleineren Stückzahlen pro Variante herzustellen.

Um das intrakorporale Gerät 40 in die Verbindungseinheit 20 einzuführen, ist es erforderlich, zuvor die Kappe 7 von der Krempe 15 abzunehmen. Für das Einführen ist es aber dank der geraden Röhre 6, 14, 2 nicht erforderlich, die Verbindungseinheit 20 vom Beatmungsgerät 30 zu lösen oder die Verbindung zwischen der Verbindungseinheit 20 und der patientenseitigen Fluidführungseinheit 11 zu trennen.

Falls das gesamte Anschlussstück 3 aus dem Mittelstück 8 herausgezogen werden würde, um bei laufender künstlicher Beatmung das intrakorporale Gerät 40 einzuführen, so wäre die gesamte Aufnahme-Öffnung geöffnet. Die Gefahr ist dann groß, dass das Beatmungsgerät 30 nicht in der Lage ist, einen ausreichend großen pneumatischen end-exspiratorischen Druck (PEEP) in der Lunge des Patienten P aufrechtzuerhalten. Daher ist das Anschlussstück 3 so ausgestaltet, dass das intrakorporale Gerät 40 sich bei eingesetztem Anschlussstück 3 einführen lässt.

Falls bei eingesetztem Anschlussstück 3 das intrakorporale Gerät 40 eingeführt werden soll, muss das flächige Element 5 durchdrungen werden. Daher ist die Durchführungs-Öffnung Ö durch das flächige Element 5 durchgeführt. Beim Einführen durchstößt das Gerät 40 diese Durchführungs-Öffnung Ö. Dies ist aufgrund der Elastizität des flächigen Elements 5 möglich, ohne das flächige Element 5 zu beschädigen, auch wenn das intrakorporale Gerät 40 breiter als die Durchführungs-Öffnung Ö ist. Solange kein intrakorporales Gerät 40 eingeführt ist, nimmt die Durchführungs-Öffnung Ö aufgrund der Elastizität des flächigen Elements 5 nur eine kleine Fläche der gebogenen Oberfläche ein, bevorzugt maximal 20 %, besonders bevorzugt maximal 10 % oder sogar maximal 5 %. Dadurch tritt auch dann, wenn die Kappe 7 von der Krempe 15 abgenommen ist, nur eine relativ geringe Menge von Fluid aus dem Strömungskanal SK durch das gerade Röhrensegment 6 nach außen aus oder dringt umgekehrt von außen in den Strömungskanal SK ein, auch wenn kein intrakorporales Gerät eingesetzt ist. Somit treten nur geringe Leckageverluste auf. Das Beatmungsgerät 30 kann daher einen ausreichenden endexspiratorischen Druck (PEEP) in der Lunge des Patienten P aufrechterhalten.

In vielen Fällen ist es nicht erforderlich, die Geometrie der Durchführungs-Öffnung Ö an die Geometrie eines einzuführenden intrakorporalen Geräts 40 anzupassen. Durch dieselbe Durchführungs-Öffnung Ö hindurch lassen sich vielmehr nacheinander verschiedene intrakorporale Geräte 40 mit unterschiedlichen Geometrien einführen und wieder herausnehmen. Weil die Durchführungs-Öffnung Ö dann, wenn kein Gerät 40 eingeführt ist, nur ein kleines Loch in der Wand des Strömungskanals SK bewirkt, ist es in vielen Fällen nicht erforderlich, nach dem Abnehmen der Kappe 7 sehr rasch das intrakorporale Gerät 40 einzuführen, um den Strömungskanal SK wieder weitgehend zu verschließen.

Möglich ist, dass die Durchführungs-Öffnung Ö von Dichtlippen oder sonstigen verformbaren Elementen umgeben ist, die eine größere Elastizität und / oder Verformbarkeit als der Rest des flächigen Elements 5 aufweisen. Beispielsweise sind die Dichtlippen aus Gummi, während der Rest des Anschlussstücks 3 aus einem härteren Kunststoff gefertigt ist. Oder das gesamte Anschlussstück 3 ist aus einem ausreichend flexiblen Material, z.B. Gummi, gefertigt. Das einzuführende intrakorporale Gerät 40 kann daher eine größere Abmessung in eine Richtung senkrecht auf der Längsachse der bereitgestellten geraden Röhre aufweisen als die Durchführungs-Öffnung Ö, und zwar dann, wenn kein intrakorporales Gerät 40 eingesetzt ist.

Bevorzugt ist die Durchführungs-Öffnung Ö länglich ausgestaltet und erstreckt sich entlang einer Längsachse. Insbesondere ist dessen maximale Abmessung mindestens doppelt so groß wie die Abmessung senkrecht auf der Längsachse. Dadurch kann die Durchführungs-Öffnung Ö besonders weit geöffnet werden, um das Gerät 40 einzuführen. In einer Ausgestaltung hat die Durchführungs-Öffnung Ö die Form eines Schlitzes. Möglich ist auch, dass die Öffnung Ö die Form eines Stockankers hat, vgl. Figur 6, oder die Form eines Sterns oder einer Ellipse oder eines Halbkreises. Diese Ausgestaltung in Verbindung mit der Elastizität des flächigen Elements 5 erleichtert es, ein intrakorporales Gerät 40 mit einer relativ großen Querschnittsfläche durch die Durchführungs-Öffnung Ö hindurch einzuführen, während gleichzeitig die Fläche der Durchführungs-Öffnung Ö gering ist, wenn kein intrakorporales Gerät 40 eingeführt ist.

In einer bevorzugten Ausgestaltung werden das Mittelstück 8, die beiden Verbindungsstücke 1 und 2 sowie das Anschlussstück 3 getrennt voneinander hergestellt. Das Anschlussstück 3 wird in das Mittelstück 8 eingesetzt. Hierbei wird das flächige Element 5 des Anschlussstücks 3 in die Aufnahme-Öffnung im Mittelstück 8 eingesetzt, so dass die gebogene Oberfläche sich bündig an die Innenwand des Mittelstücks 8 anschließt. Anschließend oder zuvor werden die Verbindungsstücke 1 und 2 auf die beiden Enden des Mittelstücks 8 aufgesetzt, sodass jeweils eine drehbare fluiddichte Verbindung hergestellt wird.

Bevorzugt werden wenigstens das Mittelstück 8 und die beiden Verbindungsstücke 1 und 2 durch ein Spritzgießverfahren hergestellt, optional auch das Anschlussstück 3. Möglich ist auch, dass das Anschlussstück 3 durch Gießen beispielsweise aus Gummi hergestellt wird, bevorzugt als ein einziges Bauteil mithilfe einer entsprechenden Gießform oder mit jeweils einer Gießform pro Variante des Anschlussstücks 3. Wie oben bereits dargelegt, erstreckt sich die Aufnahme-Öffnung im Mittelstück 8, in welche später das Anschlussstück 3 eingesetzt wird, in Fließrichtung über die gesamte Ausdehnung der äußeren Wand. Diese Ausgestaltung vermeidet Hinterschneidungen im Mittelstück 8 und erleichtert es, ein zweiteiliges Formwerkzeug für das Spritzgießen bereitzustellen und zu verwenden. In einer Ausgestaltung wird eine Hälfte einer gebogenen Röhre hergestellt und mit den beiden geraden Röhrensegmenten 13 und 14 fest und fluiddicht verbunden. Dadurch ist das Mittelstück 8 mit der Aufnahme-Öffnung hergestellt.

**Bezugszeichenliste**

| | |
|---|---|
| 1 | geräteseitiges Verbindungsstück, ist drehbar und fluiddicht mit dem geräteseitigen Röhrensegment 13 verbunden, lässt sich mittels des Konus 12 drehfest mit der geräteseitigen Fluidführungseinheit 10 verbinden |
| 2 | patientenseitiges Verbindungsstück, ist drehbar und fluiddicht mit dem patientenseitigen Röhrensegment 14 verbunden, lässt sich drehfest mit der patientenseitigen Fluidführungseinheit 11 verbinden |
| 3 | Anschlussstück zum Anschließen eines intrakorporalen Geräts 40 oder zum Einführen von Medikamenten, ist in eine Aufnahme-Öffnung im Mittelstück 8 eingesetzt |
| 4 | gekrümmtes Röhrensegment des Mittelstücks 8, fest und fluiddicht mit den beiden geraden Röhrensegmenten 13 und 14 verbunden, weist eine Aufnahme-Öffnung zur Aufnahme des Anschlussstücks 3 auf |
| 5 | flächiges Element des Anschlussstücks 3, weist die gebogene Oberfläche und die Durchführungs-Öffnung Ö auf, füllt die Aufnahme-Öffnung aus |
| 6 | gerades Röhrensegment des Anschlussstücks 3, führt von der Krempe 15 zum flächigen Element 5, lässt sich durch die Kappe 7 verschließen |
| 7 | Kappe zum Verschließen des geraden Röhrensegments 6, lässt sich auf die Krempe 15 aufsetzen und wieder abnehmen |
| 8 | Mittelstück, umfasst die geraden Röhrensegmente 13 und 14 sowie das gekrümmte Röhrensegment 4, ist drehbar mit den beiden Verbindungsstücken 1 und 2 und fest mit dem Stutzen 33 verbunden, weist eine Aufnahme-Öffnung auf, in welche das Anschlussstück 3 eingreift |
| 9.1, 9.2 | gegenüberliegende Griffelemente an dem patientenseitigen Verbindungsstück 2 |
| 10 | geräteseitige Fluidführungseinheit in Form eines röhrenförmigen Konnektors, fluiddicht mit dem geräteseitigen Verbindungsstück 1 verbunden |
| 11 | patientenseitige Fluidführungseinheit in Form eines röhrenförmigen Konnektors zwischen dem patientenseitigen Verbindungsstück 2 und dem Beatmungstubus 19, fluiddicht mit dem patientenseitigen Verbindungsstück 2 verbunden |
| 12 | Konus am geräteseitigen Verbindungsstück 1, umgreift das geräteseitige gerade Röhrensegment 13 |
| 13 | geräteseitiges gerades Röhrensegment des Mittelstücks 8, drehbar mit dem geräteseitigen Verbindungsstück 1 verbunden |
| 14 | patientenseitiges gerades Röhrensegment des Mittelstücks 8, drehbar mit dem patientenseitigen Verbindungsstück 2 verbunden, umfasst die beiden Griffelemente 9.1 und 9.2 |
| 15 | Krempe des Anschlussstücks 3, schließt an das gerade Röhrensegment 6 an, weist eine Öffnung auf, die sich von der Kappe 7 verschließen lässt |
| 17 | Y-Stück, verbindet die beiden geräteseitigen Schläuche 21.1 und 21.2 mit dem Konnektor 25 |
| 19 | patientenseitige Kopplungseinheit in Form eines Messschlauchs, der bis zum Magen des Patienten P erreicht, oder in Form eines Beatmungstubus, der in die Luftröhre des Patienten P eingeführt ist, ist fluiddicht mit dem Konnektor 11 verbunden |
| 20 | erfindungsgemäße Verbindungseinheit, umfasst das geräteseitige Verbindungsstück 1, das patientenseitige Verbindungsstück 2, das Anschlussstück 3 und das Mittelstück 8, ist fluiddicht mit den beiden |
| | Fluidführungseinheiten (Konnektoren) 10 und 11 verbunden oder verbindbar |
| 21.1, 21.2 | geräteseitige Schläuche für die Inspiration bzw. für die Exspiration, mit den Verbindungselementen 22.1 bzw. 22.2 verbunden |
| 22.1, 22.2 | Verbindungselemente für eine drehbare Verbindung zwischen den geräteseitigen Schläuchen 21.1 bzw. 21.2 und dem Beatmungsgerät 30 |
| 24 | Filter gegen Mikroben und Viren, zwischen den beiden Konnektoren 10 und 25 angeordnet |
| 25 | Fluidführungseinheit in Form eines Konnektors, verbindet das Y-Stück 17 mit dem Filter 24 |
| 30 | Beatmungsgerät, umfasst das Anzeigegerät 31, ist über die beiden Verbindungselemente 22.1, 22.2 mit dem Fluidführungssystem 100 verbunden |
| 31 | Anzeigegerät des Beatmungsgeräts 30, ermöglicht die Anzeige von Vitalparametern des Patienten P, während das Beatmungsgerät 30 über das Fluidführungssystem 100 mit dem Patienten P verbunden ist |
| 32 | um laufender Vorsprung auf dem geraden Röhrensegment 6, |
| 33 | gerader Stutzen zum Aufnehmen des Anschlussstücks 3, gehört zum Mittelstück 8, fest mit dem gekrümmten Röhrensegment 4 verbunden, endet in der Aufnahme-Öffnung |
| 34 | Schlaufe zum Halten des Anschlussstücks 3 am Mittelstück 8 |
| 40 | intrakorporales Gerät, welches sich durch das Anschlussstück 3 und die patientenseitige Fluidführungssystem 11 hindurch einführen lässt |
| 100 | Fluidführungssystem, umfasst den Beatmungstubus 19, das Mittelstück 23, die beiden geräteseitigen Schläuche 21.1 und 21.2, die beiden geräteseitigen Konnektoren 10 und 25, das Y-Stück 17 und die erfindungsgemäße Verbindungseinheit 20, ist über die |
| | beiden Verbindungselemente 22.1 und 22.2 drehbar mit dem Beatmungsgerät 30 verbunden |
| Ö | Durchführungs-Öffnung in der gebogenen Oberfläche 5 zum Hindurchführen eines intrakorporalen Geräts 40 |
| P | Patient, der künstlich beatmet wird, ist über das Fluidführungssystem 100 mit dem Beatmungsgerät 30 verbunden |
| SK | teilweise gekrümmter Strömungskanal, führt durch die Verbindungseinheit 20 hindurch, wird von den Innenwänden des Mittelstücks 8 und der Verbindungsstücke 1 und 2 bereitgestellt |

## Patentansprüche

1. Verbindungseinheit (20) zum Herstellen einer Fluidverbindung zwischen einer patientenseitigen Kopplungseinheit (19) und einem medizinischen Gerät, wobei das medizinische Gerät ein Beatmungsgerät (30) oder ein Anästhesiegerät ist,
wobei die Verbindungseinheit (20)
- ein hohles patientenseitiges Verbindungsstück (2) zum Anschließen einer mit der patientenseitigen Kopplungseinheit (19) verbundenen oder verbindbaren patientenseitigen Fluidführungseinheit (11),
- ein hohles geräteseitiges Verbindungsstück (1) zum Anschließen einer mit dem medizinischen Gerät (30) verbundenen oder verbindbaren geräteseitigen Fluidführungseinheit (10, 17, 21.1, 21.2, 25),
- ein Anschlussstück (3) und
- ein hohles Mittelstück (8) mit zwei Enden
umfasst,
wobei das Anschlussstück (3)
- ein gerades Röhrensegment (6) und
- ein flächiges Element (5) mit einer gebogenen Oberfläche und einer Durchführungs-Öffnung (Ö)
umfasst,
wobei die Durchführungs-Öffnung (Ö)
- in der gebogenen Oberfläche des flächigen Elements (5) angeordnet ist und
- höchstens die Hälfte der gebogenen Oberfläche des flächigen Elements (5) einnimmt,
wobei das Mittelstück (8) ein gekrümmtes Röhrensegment (4) mit einer Wand umfasst,
wobei das eine Ende des Mittelstücks (8) mit dem patientenseitigen Verbindungsstück (2) und das andere Ende des Mittelstücks (8) mit dem geräteseitigen Verbindungsstück (1) fluiddicht verbunden ist,
wobei die Verbindungseinheit (20) eine gekrümmte Röhre (SK) bereitstellt, welche die beiden hohlen Verbindungsstücke (1, 2) und das gekrümmte Röhrensegment (4) umfasst,
wobei das Anschlussstück (3) so in eine Aufnahme-Öffnung des Mittelstücks (8) eingesetzt oder einsetzbar ist, dass bei eingesetztem Anschlussstück (3) die gebogene Oberfläche des flächigen Elements (5) einen Teil der Wand des gekrümmten Röhrensegments (4) bildet, und
wobei das gerade Röhrensegment (6) des Anschlussstücks (3) und das Mittelstück (8) zusammen eine gerade Röhre bereitstellen, welche
- durch das flächige Element (5) des eingesetzten Anschlussstücks (3) unterbrochen ist,
- durch die Durchführungs-Öffnung (Ö) hindurchführt und
- sich im Inneren des patientenseitigen Verbindungsstücks (2) fortsetzt.

2. Verbindungseinheit (20) nach Anspruch 1,
**dadurch gekennzeichnet, dass**
das Anschlussstück (3) lösbar mit dem Mittelstück (8) verbunden ist.

3. Verbindungseinheit (20) nach einem der vorhergehenden Ansprüche, **dadurch gekennzeichnet, dass**
das Anschlussstück (3) aus einem Material ist, welches eine größere Elastizität als das oder jedes Material des Mittelstücks (8) aufweist,
bevorzugt eine größere Elastizität als das oder jedes Material der restlichen Verbindungseinheit (20).

4. Verbindungseinheit (20) nach einem der vorhergehenden Ansprüche, **dadurch gekennzeichnet, dass**
die Verbindungseinheit (20) eine Kappe (7) umfasst,
mit welcher das gerade Röhrensegment (6) reversibel verschließbar ist.

5. Verbindungseinheit (20) nach einem der vorhergehenden Ansprüche, **dadurch gekennzeichnet, dass**
das flächige Element (5) mit der gebogenen Oberfläche mindestens zwei einander gegenüberliegende Dichtlippen oder eine umlaufende Dichtlippe bereitstellt,
welche die Durchführungs-Öffnung (Ö) begrenzen bzw. begrenzt.

6. Verbindungseinheit (20) nach einem der vorhergehenden Ansprüche, **dadurch gekennzeichnet, dass**
die von der Verbindungseinheit (20) bereitgestellte gekrümmte Röhre (SK) so ausgestaltet ist,
dass sie die Richtung eines durch die Röhre (SK) fließenden Fluids um einen Winkel ablenkt, der zwischen 35° und 90° liegt.

7. Verbindungseinheit (20) nach einem der vorhergehenden Ansprüche, **dadurch gekennzeichnet, dass**
die Wand des gekrümmten Röhrensegments (4), bezogen auf die Krümmungsrichtung, ein gebogenes inneres Wandsegment und ein gebogenes äußeres Wandsegment aufweist,
wobei die gebogene Oberfläche des flächigen Elements (5) das gesamte äußere Wandsegment oder einen Teil des äußeren Wandsegments bildet,
wobei bevorzugt die gebogene Oberfläche mindestens 50 %, besonders bevorzugt mindestens 80 % der Oberfläche des äußeren Wandsegments einnimmt.

8. Verbindungseinheit (20) nach einem der vorhergehenden Ansprüche, **dadurch gekennzeichnet, dass**
das Mittelstück (8) einen Stutzen (33) umfasst, der die Aufnahme-Öffnung des Mittelstücks (8) umgibt,
wobei das Anschlussstück (3) in den Stutzen (33) eingesetzt oder einsetzbar ist und
wobei bevorzugt das Anschlussstück (3) im Stutzen (33) eingerastet oder einrastbar ist.

9. Fluidführungs-Bauteil umfassend
- eine geräteseitige Fluidführungseinheit (10, 17, 21.1, 21.2, 25) und
- eine Verbindungseinheit (20) nach einem der vorhergehenden Ansprüche,
wobei das geräteseitige Verbindungsstück (1) der Verbindungseinheit (20) fluiddicht mit der geräteseitigen Fluidführungseinheit (10, 17, 21.1, 21.2, 25) verbunden oder verbindbar ist und
wobei die geräteseitige Fluidführungseinheit (10, 17, 21.1, 21.2, 25) fluiddicht mit einem medizinischen Gerät, wobei das medizinische Gerät ein Beatmungsgerät (30) oder ein Anästhesiegerät ist, verbunden oder verbindbar ist.

10. Fluidführungs-Anordnung umfassend
- ein Fluidführungs-Bauteil nach Anspruch 9 und
- eine patientenseitige Fluidführungseinheit (11),
wobei das patientenseitige Verbindungsstück (2) der Verbindungseinheit (20) fluiddicht mit der patientenseitigen Fluidführungseinheit (11) verbunden oder verbindbar ist und
wobei die patientenseitige Fluidführungseinheit (11) fluiddicht mit einer patientenseitigen Kopplungseinheit (19) verbunden oder verbindbar ist.

11. Fluidführungssystem (100) umfassend
- eine Fluidführungs-Anordnung nach Anspruch 10 und
- eine patientenseitige Kopplungseinheit (19),
wobei die patientenseitige Fluidführungseinheit (11) fluiddicht mit der patientenseitigen Kopplungseinheit (19) verbunden oder verbindbar ist.

12. Medizinische Anordnung zur Behandlung eines Patienten (P) umfassend
- ein medizinisches Gerät, wobei das medizinische Gerät ein Beatmungsgerät (30) zum Beatmen eines Patienten (P) oder ein Anästhesiegerät ist, und
- ein Fluidführungssystem (100) nach Anspruch 11,
wobei das Fluidführungssystem (100) eine Fluidverbindung zwischen der patientenseitigen Kopplungseinheit (19) und dem medizinischen Gerät (30) herstellt oder herzustellen vermag.

13. Verwendung einer Verbindungseinheit (20) nach einem der Ansprüche 1 bis 8 oder eines Fluidführungs-Bauteils nach Anspruch 9 oder einer Fluidführungs-Anordnung nach Anspruch 10 oder eines Fluidführungssystems (100) nach Anspruch 11 zum Herstellen einer Fluidverbindung zwischen einer patientenseitigen Kopplungseinheit (19) und einem medizinischen Gerät, wobei das medizinische Gerät ein Beatmungsgerät (30) oder ein Anästhesiegerät ist.

14. Verfahren zum Herstellen einer Verbindungseinheit (20) nach einem der Ansprüche 1 bis 8,
wobei das Verfahren die Schritte umfasst, dass
- das Anschlussstück (3) hergestellt wird,
- die beiden Verbindungsstücke (1, 2) hergestellt werden,
- das Mittelstück (8) dergestalt hergestellt wird, dass die Wand des Mittelstücks (8) die Aufnahme-Öffnung aufweist,
- das Anschlussstück (3) dergestalt in die Aufnahme-Öffnung eingesetzt wird, dass das flächige Element (5) des Anschlussstücks (3) die Aufnahme-Öffnung verschließt, und
- das Mittelstück (8) mit den beiden Verbindungsstücken (1, 2) fluiddicht verbunden wird.

15. Computerprogramm, welches auf einem 3D-Drucker ausführbar ist und bei der Ausführung auf dem 3D-Drucker bewirkt, dass der 3D-Drucker mindestens die beiden Verbindungsstücke (1, 2), das Anschlussstück (3) und das Mittelstück (8) einer Verbindungseinheit (20) nach einem der Ansprüche 1 bis 8 erzeugt.

16. 3D-Drucker, welcher dazu ausgestaltet ist, mindestens die beiden Verbindungsstücke (1, 2), das Anschlussstück (3) und das Mittelstück (8) einer Verbindungseinheit (20) nach einem der Ansprüche 1 bis 8 zu erzeugen, wobei der 3D-Drucker einen Speicher mit einem Computerprogramm nach Anspruch 15 umfasst.

## Claims

1. Connecting unit (20) for producing a fluid connection between a patient-side coupling unit (19) and a medical apparatus, wherein the medical apparatus is a respiratory apparatus (30) or an anaesthesia apparatus, wherein the connecting unit (20) comprises
- a hollow patient-side connecting piece (2) for attaching a patient-side fluid-conducting unit (11), which is connected or connectable to the patient-side coupling unit (19),
- a hollow apparatus-side connecting piece (1) for attaching an apparatus-side fluid-conducting unit (10, 17, 21.1, 21.2, 25), which is connected or connectable to the medical apparatus (30),
- an attachment piece (3) and
- a hollow central piece (8) with two ends,
wherein the attachment piece (3) comprises
- a rectilinear tube segment (6) and
- a planar element (5) with an arched surface and a feedthrough opening (Ö),
wherein the feedthrough opening (Ö)
- is arranged in the arched surface of the planar element (5) and
- takes up at most half of the arched surface of the planar element (5),
wherein the central piece (8) comprises a curved tube segment (4) with a wall,
wherein the one end of the central piece (8) is connected fluid-tightly to the patient-side connecting piece (2) and the other end of the central piece (8) is connected fluid-tightly to the apparatus-side connecting piece (1), wherein the connecting unit (20) provides a curved tube (SK) which comprises the two hollow connecting pieces (1, 2) and the curved tube segment (4),
wherein the attachment piece (3) is inserted or insertable into a receiving opening of the central piece (8) in such a manner that, when the attachment piece (3) is inserted, the arched surface of the planar element (5) forms part of the wall of the curved tube segment (4), and
wherein the rectilinear tube segment (6) of the attachment piece (3) and the central piece (8) together provide a rectilinear tube which
- is interrupted by the planar element (5) of the inserted attachment piece (3),
- leads through the feedthrough opening (Ö) and
- continues in the interior of the patient-side connecting piece (2).

2. Connecting unit (20) according to Claim 1,
**characterized in that**
the attachment piece (3) is releasably connected to the central piece (8).

3. Connecting unit (20) according to either of the preceding claims, **characterized in that**
the attachment piece (3) is made from a material which has a greater elasticity than the or each material of the central piece (8),
preferably a greater elasticity than the or each material of the rest of the connecting unit (20).

4. Connecting unit (20) according to one of the preceding claims, **characterized in that**
the connecting unit (20) comprises a cap (7)
with which the rectilinear tube segment (6) is reversibly closable.

5. Connecting unit (20) according to one of the preceding claims, **characterized in that**
the planar element (5) with the arched surface provides at least two mutually opposite sealing lips or a peripheral sealing lip
which delimit or delimits the feedthrough opening (Ö).

6. Connecting unit (20) according to one of the preceding claims, **characterized in that**
the curved tube (SK) provided by the connecting unit (20) is configured in such a manner
that it deflects the direction of a fluid flowing through the tube (SK) by an angle of between 35° and 90°.

7. Connecting unit (20) according to one of the preceding claims, **characterized in that**
the wall of the curved tube segment (4) has, with respect to the direction of curvature, an arched inner wall segment and an arched outer wall segment, wherein the arched surface of the planar element (5) forms the entire outer wall segment or part of the outer wall segment, wherein preferably the arched surface takes up at least 50%, particularly preferably at least 80% of the surface of the outer wall segment.

8. Connecting unit (20) according to one of the preceding claims, **characterized in that**
the central piece (8) comprises a nozzle (33) which surrounds the receiving opening of the central piece (8),
wherein the attachment piece (3) is inserted or insertable into the nozzle (33) and
wherein preferably the attachment piece (3) is latched or latchable in the nozzle (33).

9. Fluid-conducting component comprising
- an apparatus-side fluid-conducting unit (10, 17, 21.1, 21.2, 25) and
- a connecting unit (20) according to one of the preceding claims, wherein the apparatus-side connecting piece (1) of the connecting unit (20) is connected or connectable fluid-tightly to the apparatus-side fluid-conducting unit (10, 17, 21.1, 21.2, 25) and
wherein the apparatus-side fluid-conducting unit (10, 17, 21.1, 21.2, 25) is connected or connectable fluid-tightly to a medical apparatus, wherein the medical apparatus is a respiratory apparatus (30) or an anaesthesia apparatus.

10. Fluid-conducting arrangement comprising
- a fluid-conducting component according to Claim 9 and
- a patient-side fluid-conducting unit (11),
wherein the patient-side connecting piece (2) of the connecting unit (20) is connected or connectable fluid-tightly to the patient-side fluid-conducting unit (11) and
wherein the patient-side fluid-conducting unit (11) is connected or connectable fluid-tightly to a patient-side coupling unit (19).

11. Fluid-conducting system (100) comprising
- a fluid-conducting arrangement according to Claim 10 and
- a patient-side coupling unit (19),
wherein the patient-side fluid-conducting unit (11) is connected or connectable fluid-tightly to the patient-side coupling unit (19).

12. Medical arrangement for treating a patient (P), comprising
- a medical apparatus, wherein the medical apparatus is a respiratory apparatus (30) for respiration of a patient (P) or an anaesthesia apparatus, and
- a fluid-conducting system (100) according to Claim 11,
wherein the fluid-conducting system (100) produces or is capable of producing a fluid connection between the patient-side coupling unit (19) and the medical apparatus (30).

13. Use of a connecting unit (20) according to one of Claims 1 to 8 or a fluid-conducting component according to Claim 9 or a fluid-conducting arrangement according to Claim 10 or a fluid-conducting system (100) according to Claim 11 for producing a fluid connection between a patient-side coupling unit (19) and a medical apparatus, wherein the medical apparatus is a respiratory apparatus (30) or an anaesthesia apparatus.

14. Method for producing a connecting unit (20) according to one of Claims 1 to 8,
wherein the method comprises the steps that
- the attachment piece (3) is produced,
- the two connecting pieces (1, 2) are produced,
- the central piece (8) is produced in such a manner that the wall of the central piece (8) has the receiving opening,
- the attachment piece (3) is inserted into the receiving opening in such a manner that the planar element (5) of the attachment piece (3) closes the receiving opening, and
- the central piece (8) is connected fluid-tightly to the two connecting pieces (1, 2).

15. Computer program which can be executed on a 3D printer and, upon execution on the 3D printer, causes the 3D printer to produce at least the two connecting pieces (1, 2), the attachment piece (3) and the central piece (8) of a connecting unit (20) according to one of Claims 1 to 8.

16. 3D printer which is configured to produce at least the two connecting pieces (1, 2), the attachment piece (3) and the central piece (8) of a connecting unit (20) according to one of Claims 1 to 8, wherein the 3D printer comprises a memory with a computer program according to Claim 15.

## Revendications

1. Unité de liaison (20) destinée à la réalisation d'une liaison fluidique entre une unité d'accouplement (19) du côté d'un patient et un appareil médical, l'appareil médical étant un ventilateur (30) ou un appareil d'anesthésie,
l'unité de liaison (20) comportant
- une pièce de liaison (2) creuse du côté d'un patient destinée au raccordement d'une unité de guidage de fluide (11) du côté du patient reliée ou pouvant être reliée à l'unité d'accouplement (19) du côté du patient,
- une pièce de liaison (1) creuse du côté de l'appareil destinée au raccordement d'une unité de guidage de fluide (10, 17, 21.1, 21.2, 25) du côté de l'appareil reliée ou pouvant être reliée à l'appareil médical (30),
- une pièce de raccordement (3) et
- une pièce centrale (8) creuse dotée de deux extrémités,
la pièce de raccordement (3) comportant
- un segment de tube (6) rectiligne et
- un élément (5) plan doté d'une surface courbée et d'une ouverture de passage (Ö),
l'ouverture de passage (Ö)
- étant disposée dans la surface courbée de l'élément (5) plan et
- occupant au maximum la moitié de la surface courbée de l'élément (5) plan,
la pièce centrale (8) comportant un segment de tube (4) courbe doté d'une paroi,
l'une des extrémités de la pièce centrale (8) étant reliée fluidiquement à la pièce de liaison (2) du côté du patient et l'autre extrémité de la pièce centrale (8) étant reliée fluidiquement à la pièce de liaison (1) du côté de l'appareil, l'unité de liaison (20) fournissant un tube courbe (SK), lequel comporte les deux pièces de liaison (1, 2) creuses et le segment de tube (4) courbe,
la pièce de liaison (3) étant insérée ou pouvant être insérée dans une ouverture de réception de la pièce centrale (8) de telle sorte que, lorsque la pièce de raccordement (3) est insérée, la surface courbée de l'élément (5) plan forme une partie de la paroi du segment de tube (4) courbe, et
le segment de tube (6) rectiligne de la pièce de raccordement (3) et la pièce centrale (8) fournissant conjointement un tube rectiligne, lequel
- est interrompu par l'élément (5) plan de la pièce de raccordement (3) insérée,
- est guidé à travers l'ouverture de passage (Ö) et
- se prolonge dans l'intérieur de la pièce de raccordement (2) du côté du patient.

2. Unité de liaison (20) selon la revendication 1,
**caractérisée en ce que**
la pièce de raccordement (3) est reliée de manière amovible à la pièce centrale (8).

3. Unité de liaison (20) selon l'une des revendications précédentes, **caractérisée en ce que**
la pièce de raccordement (3) est constituée d'un matériau, lequel présente une plus grande élasticité que le ou chaque matériau de la pièce centrale (8),
de préférence une plus grande élasticité que le ou chaque matériau du reste de l'unité de liaison (20).

4. Unité de liaison (20) selon l'une des revendications précédentes, **caractérisée en ce que**
l'unité de liaison (20) comporte un capuchon (7),
à l'aide duquel le segment de tube (6) rectiligne peut être fermé de manière réversible.

5. Unité de liaison (20) selon l'une des revendications précédentes, **caractérisée en ce que**
l'élément (5) plan doté de la surface courbée fournit au moins deux lèvres d'étanchéité en regard l'une de l'autre ou une lèvre d'étanchéité périphérique, laquelle/lesquelles entoure ou entourent l'ouverture de passage (Ö).

6. Unité de liaison (20) selon l'une des revendications précédentes, **caractérisée en ce que**
le tube courbe (SK) fourni par l'unité de liaison (20) est configuré de telle sorte
qu'il dévie la direction d'un fluide s'écoulant à travers le tube (SK) d'un angle qui est compris entre 35° et 90°.

7. Unité de liaison (20) selon l'une des revendications précédentes, **caractérisée en ce que**
la paroi du segment de tube (4) courbe présente, par rapport à la direction de courbure, un segment de paroi interne courbé et un segment de paroi externe courbé,
la surface courbée de l'élément (5) plan formant tout le segment de paroi externe ou une partie du segment de paroi externe, la surface courbée occupant de préférence au moins 50 %, de manière particulièrement préférée au moins 80 % de la surface du segment de paroi externe.

8. Unité de liaison (20) selon l'une des revendications précédentes, **caractérisée en ce que**
la pièce centrale (8) comporte une tubulure (33) qui entoure l'ouverture de réception de la pièce centrale (8),
la pièce de raccordement (3) étant insérée ou pouvant être insérée dans la tubulure (33) et,
de préférence, la pièce de raccordement (3) étant encliquetée ou pouvant être encliquetée dans la tubulure (33).

9. Composant de guidage de fluide comportant
- une unité de guidage de fluide (10, 17, 21.1, 21.2, 25) du côté de l'appareil et
- une unité de liaison (20) selon l'une des revendications précédentes, la pièce de liaison (1) du côté de l'appareil de l'unité de liaison (20) étant reliée ou pouvant être reliée de manière étanche aux fluides à l'unité de guidage de fluide (10, 17, 21.1, 21.2, 25) du côté de l'appareil et
l'unité de guidage de fluide (10, 17, 21.1, 21.2, 25) du côté de l'appareil étant reliée ou pouvant être reliée de manière étanche aux fluides à un appareil médical, l'appareil médical étant un ventilateur (30) ou un appareil d'anesthésie.

10. Agencement de guidage de fluide comportant
- un composant de guidage de fluide selon la revendication 9 et
- une unité de guidage de fluide (11) du côté du patient,
la pièce de liaison (2) du côté du patient de l'unité de liaison (20) étant reliée ou pouvant être reliée de manière étanche aux fluides à l'unité de guidage de fluide (11) du côté du patient et
l'unité de guidage de fluide (11) du côté du patient étant reliée ou pouvant être reliée de manière étanche aux fluides à une unité d'accouplement (19) du côté du patient.

11. Système de guidage de fluide (100) comportant
- un agencement de guidage de fluide selon la revendication 10 et
- une unité d'accouplement (19) du côté du patient,
l'unité de guidage de fluide (11) du côté du patient étant reliée ou pouvant être reliée de manière étanche aux fluides à l'unité d'accouplement (19) du côté du patient.

12. Agencement médical servant au traitement d'un patient (P), comportant
- un appareil médical, l'appareil médical étant un ventilateur (30) servant à la ventilation d'un patient (P) ou un appareil d'anesthésie, et
- un système de guidage de fluide (100) selon la revendication 11,
le système de guidage de fluide (100) réalisant ou permettant de réaliser une liaison fluidique entre l'unité d'accouplement (19) du côté du patient et l'appareil médical (30).

13. Utilisation d'une unité de liaison (20) selon l'une des revendications 1 à 8 ou d'un composant de guidage de fluide selon la revendication 9 ou d'un agencement de guidage de fluide selon la revendication 10 ou d'un système de guidage de fluide (100) selon la revendication 11 pour la réalisation d'une liaison fluidique entre une unité d'accouplement (19) du côté du patient et un appareil médical, l'appareil médical étant un ventilateur (30) ou un appareil d'anesthésie.

14. Procédé de réalisation d'une unité de liaison (20) selon l'une des revendications 1 à 8,
le procédé comportant les étapes selon lesquelles
- la pièce de raccordement (3) est réalisée,
- les deux pièces de liaison (1, 2) sont réalisées,
- la pièce centrale (8) est réalisée de telle sorte que la paroi de la pièce centrale (8) comprend l'ouverture de réception,
- la pièce de raccordement (3) est insérée dans l'ouverture de réception de telle sorte que l'élément (5) plan de la pièce de raccordement (3) ferme l'ouverture de réception, et
- la pièce centrale (8) est reliée de manière étanche aux fluides aux deux pièces de liaison (1, 2).

15. Programme informatique, lequel peut être exécuté sur une imprimante 3D et, lors de l'exécution sur l'imprimante 3D, provoque la production, par l'imprimante 3D, d'au moins les deux pièces de liaison (1, 2), de la pièce de raccordement (3) et de la pièce centrale (8) d'une unité de liaison (20) selon l'une des revendications 1 à 8.

16. Imprimante 3D, laquelle est configurée pour produire au moins les deux pièces de liaison (1, 2), la pièce de raccordement (3) et la pièce centrale (8) d'une unité de liaison (20) selon l'une des revendications 1 à 8, l'imprimante 3D comportant une mémoire dotée d'un programme informatique selon la revendication 15.
